(19) Europäisches Patentamt
European Patent Office
Office européen des brevets

(11) Publication number: 0 017 484
B1

(12) # EUROPEAN PATENT SPECIFICATION

(45) Date of publication of patent specification: 06.04.83

(51) Int. Cl.³: C 07 D 233/50, A 61 K 31/415

(21) Application number: 80301061.0

(22) Date of filing: 02.04.80

(54) 2-Imidazoline derivatives, process for the preparation thereof and the pharmaceutical composition of the same.

(30) Priority: 03.04.79 GB 7911537

(43) Date of publication of application: 15.10.80 Bulletin 80/21

(45) Publication of the grant of the patent: 06.04.83 Bulletin 83/14

(84) Designated Contracting States: AT BE CH DE FR GB IT LI NL SE

(56) References cited: GB - A - 889 706

(73) Proprietor: Fujisawa Pharmaceutical Co., Ltd.
3, Doshomachi 4-chome Higashi-ku
Osaka-shi, Osaka 541 (JP)

(72) Inventor: Ueda, Ukuo
No. 4-13-36, Kumanocho
Toyonaka (JP)
Inventor: Matsuo, Masaaki
No. 5-4-12, Nakasakurazuda
Toyonaka (JP)
Inventor: Taniguchi, Kiyoshi
No. 3-2-3-1006, Nagarashigashi
Oyodoku Osaka (JP)
Inventor: Katsura, Yousuke
No. 3-9-19, Uenonishi
Toyonaka (JP)

(74) Representative: Pennant, Pyers et al,
Stevens, Hewlett & Perkins 5 Quality Court
Chancery Lane
London, WC2A 1HZ (GB)

EP 0 017 484 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

2-Imidazoline derivatives, process for the preparation thereof and the pharmaceutical composition of the same

This invention relates to 2-imidazoline derivatives. More particularly, it relates to new 2-imidazoline derivatives which have antihypertensive, anti-inflammatory, analgesic and anti-ulcer activities, to processes for the preparation thereof, and to pharmaceutical composition comprising the same for therapeutical treatment of hypertensive, inflammatory and gastrointestinal disorder and for relief from pain of various origins in human beings.

2-Imidazoline derivatives having the general formula

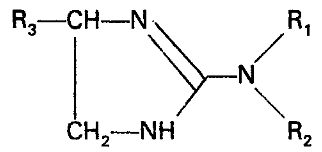

wherein $R_1$ is an aromatic or cycloaliphatic radical when $R_2$ is hydrogen or $R_1$ and $R_2$ are the same 1—4C alkyl, and $R_3$ is hydrogen or methyl, are disclosed in British Patent Specification No. 889706 as having fungicidal activity in plants. This document neither discloses nor suggests any pharmaceutical activity for these 2-imidazoline derivatives. We have discovered a class of 2-imidazoline derivatives having antihypertensive, anti-inflammatory, analgesic and anti-ulcer activities in the human body.

Accordingly, one object of this invention is to provide new and useful 2-imidazoline derivatives.

Another object of this invention is to provide processes for the preparation of 2-imidazoline derivatives.

A further object of this invention is to provide useful pharmaceutical composition comprising said 2-imidazoline derivatives as antihypertensive, anti-inflammatory, analgesic and anti-ulcer agents.

The present invention provides novel imidazoline derivatives of the formula (I):

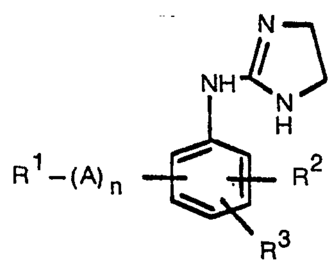

(I)

wherein $R^1$ is phenyl or naphthyl which may have 1 to 5 substituent(s) selected from halogen, 1—6C alkyl, 1—6C alkoxy, 1—6C alkanesulfonamido, mono-(or di- or tri)-halo-(1—6C)alkyl, carbamoyl, hydroxy, nitro, amino, cyano, sulfamoyl, N,N-di(1—6C)alkylsulfamoyl and di(1—6C)alkylamino which may form a 3 to 7-membered ring with or without an oxygen or another nitrogen atom;

$R^2$ and $R^3$ are each hydrogen, halogen, 1—6C alkyl, 1—6C alkoxy, 1—6C alkanesulfonamido, mono- (or di- or tri)-halo(1—6C)alkyl, carbamoyl, hydroxy, nitro, amino, cyano, sulfamoyl, N,N-di(1—6C)alkylsulfamoyl or di(1—6C)-alkylamino which may form a 3 to 7-membered ring with or without an oxygen or nitrogen atom;

A is —O—, —S— or $—CH_2—$; and

n is 0 or 1

and pharmaceutically acceptable salt thereof.

As to the objection compound (I), the following points are to be noted. That is, the "2-imidazoline-2-ylamino group in the objection compound (I) can be alternatively represented by its tautomeric group, i.e. "imidazolin-2-ylideneamino group, and both of the said groups are in the state of tautomeric equilibrium which can be represented by the following equilibrium:

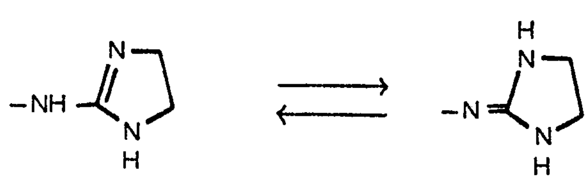

(A) (A')

These types of tautomerism between "2-imidazolin-2-ylamino" group and "imidazolin-2-ylidene-amino" group have been known in the arts, and it is obvious to a person skilled in the arts that both of the tautomeric isomers are equilibrated and lie in the reciprocally convertible state, and

accordingly it is to be understood that such isomers are included within the same category of the object compound (I) *per se*. Accordingly, the both of the tautomeric forms are clearly included within the scope of the present invention. In the present specification, the object compound (I) including the group of such tautomeric isomers is represented by using one of the expressions, i.e. "2-imidazolin-2-ylamino" group and the formula:

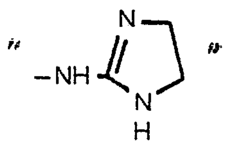

only for the convenient sake. The object compound (I) of the present invention can be prepared by the following processes.

PROCESS 1

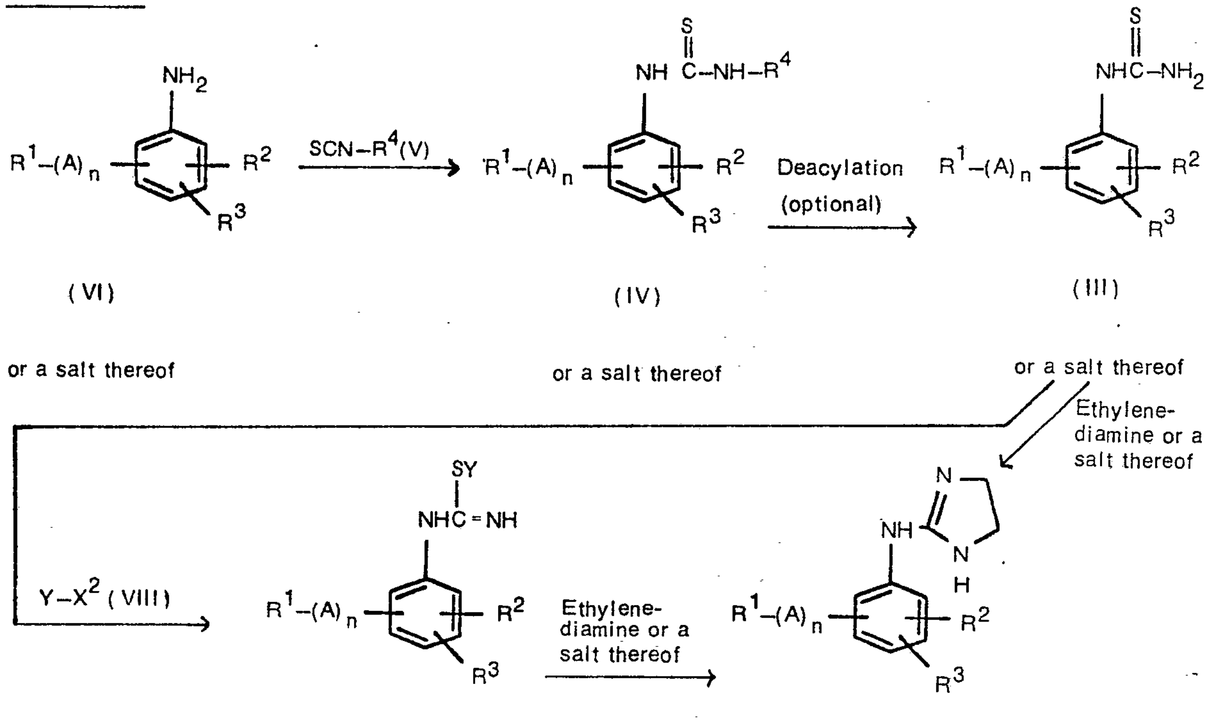


(VI) or a salt thereof

(IV) or a salt thereof

(III) or a salt thereof

(II) or a salt thereof

(I) or a salt thereof

PROCESS 2

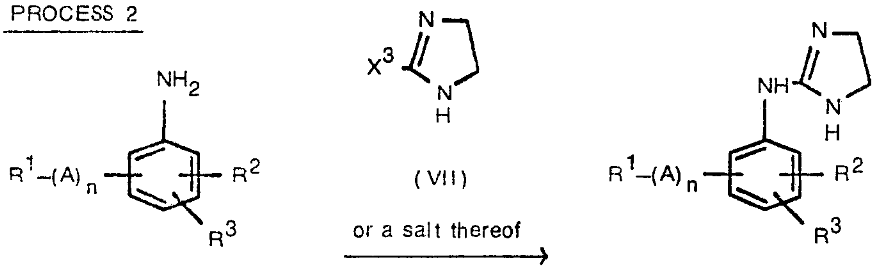


(VI) or a salt thereof

(VII)

(I) or a salt thereof

*Process 3*

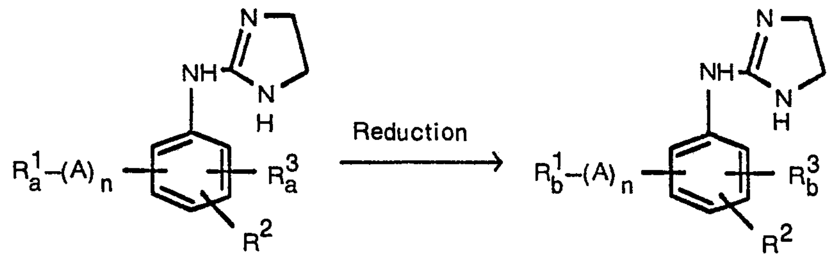


($I_b$) ($I_a$)

or a salt thereof or a salt thereof

wherein

$R^1$, $R^2$, $R^3$, A and n are each as defined above;

$R^4$ is hydrogen or acyl;

$X^2$ and $X^3$ are each an acid residue;

Y is 1—6C alkyl; and

$R^1_a$ is the same as $R^1$, and $R^3_a$ is hydrogen or nitro, provided that $R^1_a$ is aryl substituted with nitro when $R^3_a$ is hydrogen; and

$R^1_b$ is the same as $R^1$, and $R^3_b$ is hydrogen or amino, provided that $R^1_b$ is aryl substituted with amino when $R^3_b$ is hydrogen.

Suitable pharmaceutically acceptable salts of the object compounds (I) are conventional non-toxic salts such as an inorganic acid salt (e.g. hydrochloride, hydrobromide, hydroiodide, sulfate or phosphate), an organic acid salt (e.g. oxalate, maleate, fumarate, tartrate, methanesulfonate, lactate, benzenesulfonate or toluenesulfonate) or a salt with an amino acid (e.g. arginine, aspartic acid, lysine or glutamic acid).

In the above and subsequent description of the present specification, suitable examples and illustrations for the various definitions to be included within the scope of the invention are explained in details as follows.

Suitable "halogen" means fluorine, chlorine, bromine or iodine.

Examples of "1 to 6C alkyl" are methyl, ethyl, propyl, isopropyl, butyl, isobutyl, tert-butyl, pentyl and hexyl.

Examples of "1 to 6C alkoxy" are methoxy, ethoxy, propoxy, isopropoxy, butoxy, isobutoxy, pentyloxy and hexyloxy.

Examples of "1 to 6C alkenesulfonamido" are methanesulfonamido, ethanesulfonamido, propanesulfonamido, isopropanesulfonamido, butanesulfonamido and hexanesulfonamido.

Examples of "mono-(or di- or tri-)-halo(1—6C)alkyl" are chloromethyl, dibromomethyl, trifluoromethyl and dichloroethyl.

Examples of "N,N-di(1—6C)alkylsulfamoyl" are N,N-dimethylsulfamoyl, N-methyl-N-ethylsulfamoyl, N,N-dipropylsulfamoyl, N,N-diisopropylsulfamoyl, N,N-dibutylsulfamoyl, N,N-dipentylsulfamoyl and N,N-dihexylsulfamoyl.

Examples of "di(1—6C)alkylamino which may form a 3 to 7-membered ring with or without an oxygen or another nitrogen atom" are di(1—6C)alkylamino (e.g. dimethylamino, methylethylamino, dipropylamino, diisopropylamino, dibutylamino, dipentylamino and dihexylamino), 1-aziridinyl, 1-pyrrolidinyl, piperidino, morpholino, 1-imidazolidinyl, 1-piperazinyl and (1—6C)alkyl-1-piperazinyl (e.g. 4-methyl-1-piperazinyl, 4-ethyl-1-piperazinyl, 4-propyl-1-piperazinyl or 4-butyl-1-piperazinyl).

Examples of "an acid residue" are halogen, as aforementioned, azido and acyloxy (e.g. benzenesulfonyloxy or tosyloxy).

Examples of "acyl" are aliphatic acyl group, acyl group, acyl group containing an aromatic ring (hereinafter referred to as aromatic acyl) and acyl group containing a heterocyclic ring (hereinafter referred to as heterocyclic acyl).

Suitable examples of said acyl may be illustrated as follows:

Aliphatic acyl such as 1 to 18C alkanoyl (e.g. formyl, acetyl, succinyl, hexanoyl, heptanoyl or stearoyl); 1 to 18C alkoxycarbonyl (e.g. methoxycarbonyl, ethoxycarbonyl, t-butoxycarbonyl, t-pentyloxycarbonyl or heptyloxycarbonyl); or 1 to 18C alkanesulfonyl (e.g. methanesulfonyl or ethanesulfonyl).

Aromatic acyl such as aroyl (e.g. benzoyl, toluoyl or naphthoyl); phenyl(1—6C)alkanoyl (e.g. phenylacetyl and phenylpropionyl); aryloxycarbonyl (e.g. phenoxycarbonyl or naphthyloxycarbonyl); phenoxy(1—6C)alkanoyl (e.g. phenoxyacetyl or phenoxypropionyl); arylglyoxyloyl (e.g.

phenylglyoxyloyl or naphthylglyoxyloyl); or arenesulfonyl (e.g. benzenesulfonyl or p-toluenesulfonyl).

Heterocyclic acyl such as heterocycliccarbonyl (e.g. thenoyl, furoyl or nicotinoyl); heterocyclic(1—6C)alkanoyl (e.g. thienylacetyl, thiazolylacetyl and tetrazolylacetyl); or heterocyclicglyoxyloyl (e.g. thiazolylglyoxyloyl or thienylglyoxyloyl.

The acyl moiety as stated above may have one or more, same or different, suitable substituent(s) such as 1—6C alkyl as aforementioned; 1—6C alkoxy (e.g. methoxy, ethoxy or propoxy); 1—6C alkylthio (e.g. methylthio or ethylthio); 1—6C alkylamino (e.g. methylamino); cyclo (3—6C)alkyl (e.g. cyclopentyl or cyclohexyl); cyclo (5—6C)alkenyl (e.g. cyclohexenyl or cyclohexadienyl); halogen as aforementioned; amino; hydroxy; cyano; nitro; carboxy, sulfo; sulfamoyl; imino; oxo; or amino (1—6C) alkyl (e.g. aminomethyl or aminoethyl).

The processes for preparing the object compounds (I) of the present invention are explained in details in the following.

*Process 1*

(1) Preparation of the intermediate (IV)

The compound (IV) or a salt thereof can be prepared by reacting the compound (VI) or a salt thereof with the isothiocyanate compound (V).

The suitable salts of the compound (VI) can be referred to those exemplified for the compound (I).

The present reaction is usually carried out in the presence of a solvent such as acetone, methanol, tetrahydrofuran, chloroform, benzene or any other solvents which do not adversely affect the reaction.

The reaction temperature is not critical and the reaction is preferably carried out at ambient temperature or under heating.

Thus obtained compound (IV) can be used in the next step reaction without isolation.

(2) Preparation of the intermediate (III)

The compound (III) or a salt thereof can be prepared by subjecting the compound (IV) wherein $R^4$ is acyl or a salt thereof to deacylation reaction.

The suitable salt of the compound (IV) can be referred to those exemplified for the compound (I).

The present deacylation reaction is carried out in accordance with a conventional method such as hydrolysis, reduction or elimination using a Lewis acid. Among these methods, hydrolysis is one of the common and preferable method, and the hydrolysis can preferably be carried out in the presence of a base or an acid.

Examples of bases are organic or inorganic bases such as tri(1 to 6C)alkylamine (e.g. trimethylamine or triethylamine), pyridine, picoline, 1,5-diazabicyclo (4,3,0)non-5-ene, 1,4-diazabicyclo(2,2,2)-octane, 1,5-diazabicyclo(5,4,0)undecene-5, alkali metal acetate (e.g. sodium acetate, potassium acetate), alkali metal 1—6C alkoxide (e.g. sodium methoxide, sodium ethoxide or potassium tert-butoxide), alkali metal hydride (e.g. sodium hydride or potassium hydride), alkali metal hydroxide (e.g. sodium hydroxide or potassium hydroxide), alkaline earth metal hydroxide (e.g. magnesium hydroxide or calcium hydroxide), alkali metal carbonate (e.g. sodium carbonate or potassium carbonate), alkaline earth metal carbonate (e.g. magnesium carbonate or calcium carbonate), or alkali metal bicarbonate (e.g. sodium bicarbonate or potassium bicarbonate).

Examples of suitable acids are organic or inorganic acids such as formic acid, trifluoroacetic acid, benzenesulfonic acid, p-toluenesulfonic acid or hydrochloric acid.

The present reaction is usually carried out in a solvent such as water, methanol or ethanol.

The reaction temperature is not critical and the reaction is preferably carried out at ambient temperature or under warming.

(3) Preparation of the intermediate (II)

The compound (II) or a salt thereof can be prepared by reacting the compound (III) or a salt thereof with the compound (VIII).

Suitable salts of the compound (III) can be referred to those exemplified for the compound (I).

The present reaction is usually carried out in a solvent such as methanol, ethanol or any other solvents which do not adversely affect the reaction.

The present reaction can be carried out in the presence of a base as aforementioned.

The reaction temperature is not critical and the reaction is preferably carried out at ambient temperature or under warming.

The obtained product can be used directly in the next step reactions without isolation.

(4) Preparation of the object compound (I)

The object compound (I) or a salt thereof can be prepared by reacting the compound (II) or (III) or a salt thereof with ethylenediamine or salt thereof.

Suitable salts of the compound (II) can be referred to those exemplified for the compound (I).

The present reaction is usually carried out in a solvent such as methanol, ethanol or any other solvents which do not adversely affect the reaction.

The reaction temperature is not critical and the reaction is preferably carried out at ambient temperature or under warming.

*Process 2*

The compound (I) or a salt thereof can be prepared by reacting the compound (VI) or a salt thereof with the compound (VII).

The present reaction is usually carried out in a solvent such as methanol, diethyl ether or any other solvents which do not adversely affect the reaction.

The present reaction can be carried out in the presence of a base as aforementioned.

The reaction temperature is not critical and the reaction is preferably carried out at ambient temperature or under warming.

*Process 3*

The compound (Ia) or its salt can be prepared by reducing the compound (Ib) or a salt thereof.

The reduction is conducted by a conventional method which can be applied for the reduction of a nitro group into an amino group, such as reduction of using a metal (e.g. iron, zinc, etc.) and an acid (e.g. hydrochloric acid, etc.); reduction in the presence of a metallic catalyst.

The metallic catalysts for the catalytic reduction include, for example, platinum catalyst (e.g. platinum wire, spongy platinum, platinum black, platinum colloid, etc.), palladium catalyst (e.g. palladium spongy, palladium black, palladium oxide, palladium on barium sulphate, palladium on barium carbonate, palladium on charcoal, palladium on silica gel, palladium colloid, etc.), nickel catalyst (e.g. reduced nickel, nickel oxide, Raney nickel, Urushibara nickel, etc.), and the like.

The present reaction is usually carried out in a solvent such as methanol, ethanol or any other solvents which do not adversely affect the reaction.

The reaction temperature is not critical and the reaction is preferably carried out under cooling, at ambient temperature or under warming.

The following pharmacological test data show that the object compound (I) of the present invention exhibits high and long lasting antihypertensive, anti-inflammatory, analgesic and anti-ulcer activities and are accordingly useful in the treatment of hypertensive, inflammatory and gastrointestinal disorder and in the relief from pain of various origins.

1. Antihypertensive activity

*Test method:*

Five Wistar rats were used per group. Each animal was immobilized in a cage sized to the body. Blood pressure was measured at the femoral artery by means of a pressure transducer and recorded as electrically integrated values of mean arterial pressure, and heart rate was determined by a pulse wave detector. Operation for the catheterization was performed under light anesthesia with ether. The test compound (10 mg/kg) was administered orally 3 hours after completion of the operation.

*Test compound:*

Compound A: 2-(2-Phenoxy-5-chlorophenyl)amino-2-imidazoline

Compound B: 2-(2-Phenoxy-5-methylphenyl)amino-2-imidazoline

*Test results:*

Mean ratios of Δ maximum decrease of blood pressure (mmHg) were shown in the following table.

TABLE 1

| Test compound | Effect (%) |
|---|---|
| Compound A | 57 |
| Compound B | 53 |

2. Anti-inflammatory activity

*Test method:*

Ten Sprague-Dawley rats were used per group. The right hind paw of the rat was injected subcutaneously under the plantar surface with 0.1 ml of carrageenin (1.5%). After 3 hours, the animals were sacrificed. The normal and edematous hind paws were cut off at the tibio-dorsal joint and weighed. The difference in the weight of edematous paw and that of normal paw was a measure of the edema. The test compound (100 mg/kg) was administered orally 60 minutes before the irritating agent. Paw swelling of treated animals was compared with that of control animals.

*Test results:*
2-(2-Phenoxy-4-methylphenyl)amino-2-imidazoline. Inhibitory effect: 48.4%.

3. Analgesic activity
*Test method:*
Ten male ddy strain mice were used for each dose. To estimate the frequency of writhing syndrome, the animals were observed from 3 to 13 minutes after an intraperitoneal injection of 0.2 ml/10 g of 0.6% acetic acid. The drugs were given orally 60 minutes before acetic acid. The frequency of writhing syndrome in the treated animals was compared with that in the non-treated control animals.

*Test results:*
The $ED_{50}$ value was calculated according to Litchfield-Wilcoxon method.
2-[2-(3,4,5-Trimethoxyphenoxy)phenyl]amino-2-imidazoline $ED_{50}$ value: 50.1 mg/kg.

4. Anti-ulcer activity
*Test method:*
Heidenhain pouches were prepared in healthy beagle dogs several weeks before the experiments. Food was withheld out but water allowed for 18 to 24 hours before each experiment. On the day of the experiment intravenous catheter was inserted with aseptic precautions. Penta gastrin was infused at 10 $\mu$g/kg/hour via the catheter to get submaximal gastric secretion. Once a plateau of the secretion was obtained, the test compound (1 mg/kg) was injected intraveneously. The volume of secretion was measured at 15 minutes intervals and the corresponding acid output was calculated in $\mu EqH^+$/15 minutes.

*Test compound:*
Compound A: 2-(2-Biphenylyl)amino-2-imidazoline
Compound B: 2-(2-Phenylthiophenyl)amino-2-imidazoline

*Test results:*
Results were calculated as maximal % reduction in gastric acid secretion and shown in the following table.

TABLE 2

| Test compound | Effect % |
|---|---|
| Compound A | 73.2% |
| Compound B | 65.2% |

As being apparent from the above test results, the object compound (I) of the present invention is useful for the antihypertensive, anti-inflammatory, analgesic and anti-ulcer medicines.

The effective ingredient may usually be administered with a dose of 0.1 mg/kg to 500 mg/kg, 1 to 4 times a day in a preparations such as tablet, granule, powder, capsule, syrup, injection, suppository and the like. However, the above dosage may be increased or decreased according to the age, weight or conditions of the patient or the administering method. The above mentioned preparations can be prepared in a conventional manner by using conventional carriers and additives.

The present invention is illustrated by the following Examples.

Preparation of the starting compounds

Preparation 1

To a solution of ammonium thiocyanate (7.2 g.) in dry acetone (70 ml.) was added benzoyl chloride (12.1 g.) with stirring at 50 to 55°C. The reaction was continued for an hour at this temperature. To the mixture containing benzoyl isothiocyanate was dropwise added a solution of 2-phenoxy-5-methoxyaniline (17.6 g.) in dry acetone (180 ml.) with stirring at room temperature. The reaction mixture was refluxed for an hour with stirring. After removal of the solvent, water was added to the residue. The precipitated crystals were collected by filtration, washed successively with water and a small amount of methanol and then dried to give 1-(2-phenoxy-5-methoxyphenyl)-3-benzoylthiourea (28.9 g.), mp. 163 to 165°C.

Preparation 2

The following compounds were obtained according to the similar manner to that of Preparation 1.

1) 1-[2-(3-Chlorophenoxy)phenyl]-3-benzoylthiourea, mp. 112 to 121°C.
2) 1-[2-(3-Chlorophenoxy)phenyl]-3-benzoylthiourea, mp. 149 to 158°C.
3) 1-[2-(2,6-Dichlorophenoxy)phenyl]-3-benzoylthiourea, mp. 200 to 205°C.
4) 1-[2-(3,4-Dichlorophenoxy)phenyl]-3-benzoylthiourea, mp. 141 to 142°C.
5) 1-[2-(3,5-Dichlorophenoxy)phenyl]-3-benzoylthiourea, mp. 134 to 135°C.
6) 1-[2-(o-Tolyloxy)phenyl]-3-benzoylthiourea, mp. 165 to 166°C.
7) 1-[2-(m-Tolyloxy)phenyl]-3-benzoylthiourea, mp. 144 to 148°C.
8) 1-[2-(p-Tolyloxy)phenyl]-3-benzoylthiourea, mp. 187.5 to 188.5°C.
9) 1-[2-(3,4,5-Trimethoxyphenoxy)phenyl]-3-benzoylthiourea, mp. 146 to 149°C.
10) 1-[2-(4-Fluorophenoxy)phenyl]-3-benzoylthiourea, mp. 137 to 140°C.
11) 1-[2-(4-Bromophenoxy)phenyl]-3-benzoylthiourea, mp. 170 to 172°C.
12) 1-[2-(4-Cyanophenoxy)phenyl]-3-benzoylthiourea, mp. 146 to 148°C.
13) 1-(2-Phenoxy-3-chlorophenyl)-3-benzoylthiourea, mp. 137 to 141°C.
14) 1-(2-Phenoxy-4-chlorophenyl)-3-benzoylthiourea, mp. 164 to 166°C.
15) 1-(2-Phenoxy-5-chlorophenyl)-3-benzoylthiourea, mp. 163 to 166°C.
16) 1-(2-Phenoxy-6-chlorophenyl)-3-benzoylthiourea, mp. 141 to 158°C.
17) 1-(2-Phenoxy-3-methylphenyl)-3-benzoylthiourea, mp. 146 to 148°C.
18) 1-(2-Phenoxy-5-methylphenyl)-3-benzoylthiourea, mp. 175 to 180°C.
19) 1-(2-Phenoxy-6-methylphenyl)-3-benzoylthiourea, mp. 141 to 145°C.
20) 1-(2-Phenoxy-5-hydroxyphenyl)-3-benzoylthiourea, mp. 183 to 186°C.
21) 1-(2-Phenoxy-5-nitrophenyl)-3-benzoylthiourea, mp. 210 to 212°C.
22) 1-(2-Phenoxy-5-methanesulfonamidophenyl)-3-benzoylthiourea, mp. 231 to 232°C.
23) 1-(2-Phenoxy-5-cyanophenyl)-3-benzoylthiourea, mp. 206 to 208°C.
24) 1-(2-Phenoxy-5-carbamoylphenyl)-3-benzoylthiourea, mp. 213 to 215°C.
25) 1-(2-Phenoxy-5-trifluoromethylphenyl)-3-benzoylthiourea, mp. 170 to 172°C.
26) 1-[2-(4-Chlorophenoxy)-5-chlorophenyl]-3-benzoylthiourea, mp. 183 to 189°C.
27) 1-[2-(4-Chlorophenoxy)-5-methylphenyl]-3-benzoylthiourea mp. 196 to 197°C.
28) 1-(2-Phenylthiophenyl)-3-benzoylthiourea, mp. 65 to 75°C.
29) 1-(2-Benzylphenyl)-3-benzoylthiourea, mp. 132 to 133°C.
30) 1-(4-Phenoxyphenyl)-3-benzoylthiourea, mp. 123 to 126°C.
31) 1-(3-Phenoxyphenyl)-3-benzoylthiourea, mp. 71 to 80°C.
32) 1-[2-(1-Naphthyloxy)phenyl]-3-benzoylthiourea, mp. 132 to 160°C.
33) 1-(2-Biphenylyl)-3-benzoylthiourea, mp. 125 to 126°C.
34) 1-[2-(4-Chlorophenyl)phenyl]-3-benzoylthiourea, mp. 120 to 122°C.
35) 1-[2-Phenyl-4-chlorophenyl]-3-benzoylthiourea, mp. 190 to 191°C.
36) 1-[2-Phenyl-5-chlorophenyl]-3-benzoylthiourea, mp. 138 to 140°C.
37) 1-(2-Phenoxy-5-dimethylaminophenyl)-3-benzoylthiourea, mp. 144 to 152°C.
38) 1-[2-(2-Fluorophenyl)phenyl]-3-benzoylthiourea, mp. 124 to 126°C.
39) 1-[2-(2-Chlorophenyl)phenyl]-3-benzoylthiourea, mp. 163 to 165°C.
40) 1-(2-Phenyl-6-chlorophenyl)-3-benzoylthiourea, mp. 197 to 200°C.
41) 1-[4-(2,3-Dimethylphenoxy)-2,6-dichlorophenyl]-3-benzoylthiourea, mp. 166 to 171°C.
42) 1-[2-(3-Fluorophenyl)phenyl]-3-benzoylthiourea, mp. 84 to 113°C.
43) 1-[2-(o-Tolyl)phenyl]-3-benzoylthiourea, mp. 141 to 146°C.
44) 1-[2-(p-Tolyl)phenyl]-3-benzoylthiourea, mp. 118 to 121°C.
45) 1-[2-(4-Fluorophenyl)phenyl]-3-benzoylthiourea, mp. 104 to 116°C.
46) 1-(2-Phenoxy-5-morpholinophenyl)-3-benzoylthiourea, mp. 176 to 184°C.
47) 1-(3-Biphenylyl)-3-benzoylthiourea, mp. 157 to 158°C.
48) 1-(2-Phenyl-3-chlorophenyl)-3-benzoylthiourea, mp. 116 to 129°C.
49) 1-[2-(4-Dimethylaminophenoxy)phenyl]-3-benzoylthiourea, mp. 203 to 208°C.
50) 1-[2-Phenoxy-5-(1-pyrrolidinyl)phenyl]-3-benzoylthiourea, mp. 157 to 165°C.
51) 1-[2-(m-Tolyl)phenyl]-3-benzoylthiourea, mp. 102 to 106°C.
52) 1-[2-(2-Nitrophenyl)phenyl]-3-benzoylthiourea, mp. 79 to 94°C.
I.R. (Nujol): 3420, 1675 $cm^{-1}$
53) 1-[2-(2,4-Difluorophenyl)phenyl]-3-benzoylthiourea,
I.R. (Film): 3415, 3220, 3155, 3040, 3025, 1670 $cm^{-1}$
54) 1-[2-Phenoxy-5-(N,N-dimethylsulfamoyl)phenyl]-3-benzoylthiourea, mp. 140 to 145°C.
55) 1-[2-(2,6-Difluorophenyl)phenyl]-3-benzoylthiourea, mp. 145 to 155°C.
56) 1-(2-Phenyl-5-methylphenyl)-3-benzoylthiourea, mp. 134 to 136°C.
57) 1-[2-(2-Methoxyphenyl)phenyl]-3-benzoylthiourea, mp. 139 to 144°C.
58) 1-[2-(2-Trifluoromethylphenyl)phenyl]-3-benzoylthiourea, mp. 139 to 145°C.
59) 1-[2-(2-Dimethylaminophenyl)phenyl]-3-benzoylthiourea, mp. 154 to 164°C.
60) 1-[2-(3-Chloro-4-sulfamoylphenoxy)phenyl]-3-benzoylthiourea.
I.R. (Nujol): 3370, 3245, 1670, 1375, 1180 $cm^{-1}$

Preparation 3

A suspension of 1-(2-phenoxy-6-chlorophenyl)-3-benzoylthiourea (8.6 g.) in a solution of sodium hydroxide (2.0 g.) in water (50 ml.) was refluxed with stirring for 30 minutes. The reaction mixture was cooled acidified with conc. hydrochloric acid and extracted with chloroform (200 ml.). The extract was washed successively with 4% aqueous solution of ammonia (twice) and a saturated aqueous solution of sodium chloride, dried over magnesium sulfate and then evaporated under reduced pressure. To the residual oil, a mixture of n-hexan and petroleum ether was added. The precipitated crystals were collected by filtration and dried to give 1-(2-phenoxy-6-chlorophenyl)thiourea (5.8 g.), mp 107 to 120°C.

Preparation 4

A solution of 1-(2-phenoxy-5-methoxyphenyl)-3-benzoylthiourea (28.6 g.) in a solution of potassium hydroxide (4.2 g.) in methanol (290 ml.) was stirred for 10 minutes at 50—60°C. The reaction mixture was cooled and then evaporated. The residue was washed with water and dried to give 1-(2-phenoxy-5-methoxyphenyl)thiourea (21.0 g.), mp. 143 to 167°C.

Preparation 5

The following compounds were obtained according to the similar manner to those of Preparation 3 and 4.

1) 1-(2-Phenoxyphenyl)thiourea, mp. 122 to 125°C.
2) 1-[2-(2-Chlorophenoxy)phenyl]thiourea, mp. 125 to 128°C.
3) 1-[2-(3-Chlorophenoxy)phenyl]thiourea, mp. 102 to 107°C.
4) 1-[2-(4-Chlorophenoxy)phenyl]thiourea, mp. 139 to 147°C.
5) 1-[2-(2,6-Dichlorophenoxy)phenyl]thiourea, mp. 186 to 190°C.
6) 1-[2-(3,4-Dichlorophenoxy)phenyl]thiourea, mp. 147 to 150°C.
7) 1-[2-(3,5-Dichlorophenoxy)phenyl]thiourea, mp. 148 to 150°C.
8) 1-[2-(o-Tolyloxy)phenyl]thiourea, mp. 135 to 136°C.
9) 1-[2-(m-Tolyloxy)phenyl]thiourea, mp. 104 to 106°C.
10) 1-[2-(p-Tolyloxy)phenyl]thiourea, mp. 154 to 155°C.
11) 1-[2-(3,4,5-trimethoxyphenoxy)phenyl]thiourea, mp. 154 to 158°C.
12) 1-[2-(4-Fluorophenoxy)phenyl]thiourea, mp. 139 to 141°C.
13) 1-[2-(4-Bromophenoxy)phenyl]thiourea, mp. 155 to 157°C.
14) 1-[2-(4-Cyanophenoxy)phenyl]thiourea, mp. 159 to 161°C.
15) 1-(2-Phenoxy-3-chlorophenyl)thiourea, mp. 133 to 142°C.
16) 1-(2-Phenoxy-4-chlorophenyl)thiourea, mp. 137 to 138°C.
17) 1-(2-Phenoxy-5-chlorophenyl)thiourea, mp. 140 to 145°C.
18) 1-(2-Phenoxy-3-methylphenyl)thiourea, mp. 141 to 145°C.
19) 1-(2-Phenoxy-4-methylphenyl)thiourea, mp. 131 to 133°C.
20) 1-(2-Phenoxy-5-methylphenyl)thiourea, mp. 161 to 163°C.
21) 1-(2-Phenoxy-6-methylphenyl)thiourea, mp. 137 to 142°C.
22) 1-(2-Phenoxy-5-hydroxyphenyl)thiourea, mp. 174 to 176°C.
23) 1-(2-Phenoxy-5-nitrophenyl)thiourea, mp. 147 to 157°C.
24) 1-(2-Phenoxy-5-methanesulfonamidophenyl)thiourea, mp. 191 to 195°C.
25) 1-(2-Phenoxy-5-cyanophenyl)thiourea, mp. 187 to 189°C.
26) 1-(2-Phenoxy-5-carbamoylphenyl)thiourea, mp. 195 to 196°C.
27) 1-(2-Phenoxy-5-trifluoromethylphenyl)thiourea, mp. 159 to 160°C.
28) 1-[2-(4-Chlorophenoxy)-5-chlorophenyl]thiourea, mp. 149 to 165°C.
29) 1-[2-(4-Chlorophenoxy)-5-methylphenyl]thiourea, mp. 108 to 113°C.
30) 1-(2-Phenylthiophenyl)thiourea, mp. 99 to 103°C.
31) 1-(2-Benzylphenyl)thiourea, mp. 125 to 126°C.
32) 1-(4-Phenoxyphenyl)thiourea, mp. 181 to 184°C.
33) 1-(3-Phenoxyphenyl)thiourea, mp. 98 to 120°C.
34) 1-[2-(1-Naphthyloxyphenyl]thiourea, mp. 172 to 179°C.
35) 1-(2-Biphenylyl)thiourea, mp. 184 to 185°C.
36) 1-[2-(4-Chlorophenyl)phenyl]thiourea, mp. 199 to 201°C.
37) 1-[2-Phenyl-4-chlorophenyl]thiourea, mp. 205 to 206°C.
38) 1-[2-Phenyl-5-chlorophenyl]thiourea, mp. 187 to 189°C.
39) 1-(2-Phenoxy-5-dimethylaminophenyl)thiourea, mp. 163 to 164°C.
40) 1-[2-(2-Fluorophenyl)phenyl]thiourea, mp. 195 to 197°C.
41) 1-[2-(2-Chlorophenyl)phenyl]thiourea, mp. 190 to 192°C.
42) 1-(2-Phenyl-6-chlorophenyl)thiourea, mp. 171 to 176°C.
43) 1-[4-(2,3-Dimethylphenoxy)-2,6-dichlorophenyl]thiourea, mp. 93 to 113°C.
44) 1-[2-(3-Fluorophenyl)phenyl]thiourea, mp. 159 to 162°C.
45) 1-[2-(o-Tolyl)phenyl]thiourea, mp. 142 to 144°C.

46) 1-[2-(p-Tolyl)phenyl]thiourea, mp. 193 to 195°C.
47) 1-[2-(4-Fluorophenyl)phenyl]thiourea, mp. 158 to 161°C.
48) 1-(2-Phenoxy-5-sulfamoylphenyl)thiourea, mp. 97 to 102°C.
49) 1-(2-Phenoxy-5-morpholinophenyl)thiourea, mp. 189 to 193°C.
50) 1-(3-Biphenylyl)thiourea, mp. 183 to 185°C.
51) 1-(2-Phenyl-3-chlorophenyl)thiourea, mp. 186 to 188°C.
52) 1-[2-(4-Dimethylaminophenoxy)phenyl]thiourea, mp. 191 to 194°C.
53) 1-[2-Phenoxy-5-(1-pyrrolidinyl)phenyl]thiourea, mp. 192 to 196°C.
54) 1-[2-(m-Tolyl)phenyl]thiourea, mp. 128 to 136°C.
55) 1-[2-(2-Nitrophenyl)phenyl]thiourea, mp. 186 to 194°C.
56) 1-[2-(2,4-Difluorophenyl)phenyl]thiourea, mp. 153 to 157°C.
57) 1-[2-Phenoxy-5-(N,N-dimethylsulfamoyl)phenyl]thiourea, mp. 153 to 157°C.
58) 1-[2-(2,6-Difluorophenyl)phenyl]thiourea, mp. 232 to 234°C.
59) 1-(2-Phenyl-5-methylphenyl)thiourea, mp. 187 to 192°C.
60) 1-[2-(2-Methoxyphenyl)phenyl]thiourea, mp. 154 to 158°C.
61) 1-[2-(2-Trifluoromethylphenyl)phenyl]thiourea, mp. 186 to 187°C.
62) 1-[2-(2-Dimethylaminophenyl)phenyl]thiourea, mp. 151 to 154°C.
63) 1-[2-(3-Chloro-4-sulfamoylphenoxy)phenyl]thiourea
I.R. (Film): 3325, 3200, 1350, 1170 $cm^{-1}$

Preparation 6

To a solution of ammonium thiocyanate (4.55 g.) in dry acetone (100 ml.) was added benzoyl chloride (7.4 g.) over a period of 5 minutes with stirring at 50°C. The mixture was refluxed with stirring for 40 minutes. To the resulting mixture containing benzoyl isothiocyanate was dropwise added a solution of 2-phenoxyaniline (7.2 g.) in dry acetone (50 ml.) over a period of 10 minutes with stirring at 50°C and then the mixture was refluxed with stirring for 30 minutes. After removal of the solvent, water (100 ml.) was added. The precipitates containing 1-(2-phenoxyphenyl)-3-benzoylthiourea were collected by filtration and washed with water. The precipitates were added to a mixture of 10% aqueous solution of sodium hydroxide (100 ml.) and methanol (80 ml.) with stirring at 50°C and then stirring was continued for 30 minutes at this temperature. The reaction mixture was chilled to precipitate crystals, which were collected by filtration, washed with water and then dried to give 1-(2-phenoxyphenyl)thiourea (8.5 g.), mp. 122 to 125°C.

Preparation 7

The following compounds were obtained according to the similar manner to that of Preparation 6.

1) 1-[2-(2-Chlorophenoxy)phenyl]thiourea, mp. 125 to 128°C.
2) 1-[2-(3-Chlorophenoxy)phenyl]thiourea, mp. 102 to 107°C.
3) 1-[2-(4-Chlorophenoxy)phenyl]thiourea, mp. 139 to 147°C.
4) 1-[2-(2,6-Dichlorophenoxy)phenyl]thiourea, mp. 186 to 190°C.
5) 1-[2-(3,4-Dichlorophenoxy)phenyl]thiourea, mp. 147 to 150°C.
6) 1-[2-(3,5-Dichlorophenoxy)phenyl]thiourea, mp. 148 to 150°C.
7) 1-[2-(o-Tolyloxy)phenyl]thiourea, mp. 135 to 136°C.
8) 1-[2-(m-Tolyloxy)phenyl]thiourea, mp. 104 to 106°C.
9) 1-[2-(p-Tolyloxy)phenyl]thiourea, mp. 154 to 155°C.
10) 1-[2-(3,4,5-Trimethoxyphenoxy)phenyl]thiourea, mp. 154 to 158°C.
11) 1-[2-(4-Fluorophenoxy)phenyl]thiourea, mp. 139 to 141°C.
12) 1-[2-(4-Bromophenoxy)phenyl]thiourea, mp. 155 to 157°C.
13) 1-[2-(4-Cyanophenoxy)phenyl]thiourea, mp. 159 to 161°C.
14) 1-(2-Phenoxy-3-chlorophenyl)thiourea, mp. 133 to 142°C.
15) 1-(2-Phenoxy-4-chlorophenyl)thiourea, mp. 137 to 138°C.
16) 1-(2-Phenoxy-5-chlorophenyl)thiourea, mp. 140 to 145°C.
17) 1-(2-Phenoxy-6-chlorophenyl)thiourea, mp. 107 to 120°C.
18) 1-(2-Phenoxy-3-methylphenyl)thiourea, mp. 141 to 145°C.
19) 1-(2-Phenoxy-4-methylphenyl)thiourea, mp. 131 to 133°C.
20) 1-(2-Phenoxy-5-methylphenyl)thiourea, mp. 161 to 163°C.
21) 1-(2-Phenoxy-6-methylphenyl)thiourea, mp. 137 to 142°C.
22) 1-(2-Phenoxy-5-hydroxyphenyl)thiourea, mp. 174 to 176°C.
23) 1-(2-Phenoxy-5-methoxyphenyl)thiourea, mp. 143 to 167°C.
24) 1-(2-Phenoxy-5-nitrophenyl)thiourea, mp. 147 to 157°C.
25) 1-(2-Phenoxy-5-methanesulfonamidophenyl)thiourea, mp. 191 to 195°C.
26) 1-(2-Phenoxy-5-cyanophenyl)thiourea, mp. 187 to 189°C.
27) 1-(2-Phenoxy-5-carbamoylphenyl)thiourea, mp. 195 to 196°C.
28) 1-(2-Phenoxy-5-trifluoromethylphenyl)thiourea, mp. 159 to 160°C.
29) 1-[2-(4-Chlorophenoxy)-5-chlorophenyl]thiourea, mp. 149 to 165°C.

30) 1-[2-(4-Chlorophenoxy)-5-methylphenyl]thiourea, mp. 108 to 113°C.
31) 1-(2-Phenylthiophenyl)thiourea, mp. 99 to 103°C.
32) 1-(2-Benzylphenyl)thiourea, mp. 125 to 126°C.
33) 1-(4-Phenoxyphenyl)thiourea, mp. 181 to 184°C.
34) 1-(3-Phenoxyphenyl)thiourea, mp. 98 to 120°C.
35) 1-[2-(1-Naphthyloxy)phenyl]thiourea, mp. 172 to 179°C.
36) 1-(2-Biphenylyl)thiourea, mp. 184 to 185°C.
37) 1-[2-(4-Chlorophenyl)phenyl]thiourea, mp. 199 to 201°C.
38) 1-[2-Phenyl-4-chlorophenyl]thiourea, mp. 205 to 206°C.
39) 1-[2-Phenyl-5-chlorophenyl]thiourea, mp. 187 to 189°C.
40) 1-(2-Phenoxy-5-dimethylaminophenyl)thiourea, mp. 163 to 164°C.
41) 1-[2-(2-Fluorophenyl)phenyl]thiourea, mp. 195 to 197°C.
42) 1-[2-(2-Chlorophenyl)phenyl]thiourea, mp. 190 to 192°C.
43) 1-(2-Phenyl-6-chlorophenyl)thiourea, mp. 171 to 176°C.
44) 1-[4-(2,3-Dimethylphenoxy)-2,6-dichlorophenyl]thiourea, mp. 93 to 113°C.
45) 1-[2-(3-Fluorophenyl)phenyl]thiourea, mp. 159 to 162°C.
46) 1-[2-(o-Tolyl)phenyl]thiourea, mp. 142 to 144°C.
47) 1-[2-(p-Tolyl)phenyl]thiourea, mp. 193 to 195°C.
48) 1-[2-(4-Fluorophenyl)phenyl]thiourea, mp. 158 to 161°C.
49) 1-(2-Phenoxy-5-sulfamoylphenyl)thiourea, mp. 97 to 102°C.
50) 1-(2-Phenoxy-5-morpholinophenyl)thiourea, mp. 189 to 193°C.
51) 1-(3-Biphenylyl)thiourea, mp. 183 to 185°C.
52) 1-(2-Phenyl-3-chlorophenyl)thiourea, mp. 186 to 188°C.
53) 1-[2-(4-Dimethylaminophenoxy)phenyl]thiourea, mp. 191 to 194°C.
54) 1-[2-Phenoxy-5-(1-pyrrolidinyl)phenyl]thiourea, mp. 192 to 196°C.
55) 1-[2-(m-Tolyl)phenyl]thiourea, mp. 128 to 136°C.
56) 1-[2-(2-Nitrophenyl)phenyl]thiourea, mp. 186 to 194°C.
57) 1-[2-(2,4-Difluorophenyl)phenyl]thiourea, mp. 153 to 157°C.
58) 1-[2-Phenoxy-5-(N,N-dimethylsulfamoyl)phenyl]thiourea, mp. 153 to 157°C.
59) 1-[2-(2,6-Difluorophenyl)phenyl]thiourea, mp. 232 to 234°C.
60) 1-(2-Phenyl-5-methylphenyl)thiourea, mp. 187 to 192°C.
61) 1-[2-(2-Methoxyphenyl)phenyl]thiourea, mp. 154 to 158°C.
62) 1-[2-(2-Trifluoromethylphenyl)phenyl]thiourea, mp. 186 to 187°C.
63) 1-[2-(2-Dimethylaminophenyl)phenyl]thiourea, mp. 151 to 154°C.
64) 1-[2-(3-Chloro-4-sulfamoylphenoxy)phenyl]thiourea
I.R. (Film): 3325, 3200, 1350, 1170 $cm^{-1}$

Preparation 8

A solution of 1-(2-phenoxyphenyl)thiourea (9.6 g.) and methyl iodide (5.6 g.) in methanol (120 ml.) was refluxed for 1.5 hours. The reaction mixture was evaporated under reduced pressure. To the residue diethyl ether was added. The precipitates were collected by filtration and dried to give 1-(2-phenoxyphenyl)-2-methylisothiourea hydroiodide (14.7 g.), mp. 143 to 147°C.

Preparation 9

The following compounds were obtained according to the similar manner to that of Preparation 8.

1) 1-[2-(2-Chlorophenoxy)phenyl]-2-methylisothiourea hydroiodide, mp. 43 to 53°C.
2) 1-[2-(3-Chlorophenoxy)phenyl]-2-methylisothiourea hydroiodide, mp. 135 to 139°C.
3) 1-[2-(4-Chlorophenoxy)phenyl]-2-methylisothiourea hydroiodide, mp. 57 to 77°C.
4) 1-[2-(2,6-Dichlorophenoxy)phenyl]-2-methylisothiourea hydroiodide, mp. 78 to 115°C.
5) 1-[2-(o-Tolyloxy)phenyl]-2-methylisothiourea hydroiodide.
I.R. (KBr): 3300, 3180, 3100, 1630, 1225 $cm^{-1}$
6) 1-[2-(m-Tolyloxy)phenyl]-2-methylisothiourea hydroiodide.
I.R. (KBr): 3310, 3210, 3120, 1635, 1265, 1205 $cm^{-1}$
7) 1-[2-(p-Tolyloxy)phenyl]-2-methylisothiourea hydroiodide.
I.R. (Nujol): 3305, 3325, 1630, 1210 $cm^{-1}$
8) 2-[2-(3,4,5-Trimethoxyphenoxy)phenyl]-2-methylisothiourea hydroiodide, mp. 183 to 190°C.
9) 1-[2-(4-Fluorophenoxy)phenyl]-2-methylisothiourea hydroiodide.
I.R. (KBr): 3300, 1625, 1210 $cm^{-1}$
10) 1-[2-(4-Bromophenoxy)phenyl]-2-methylisothiourea hydroiodide.
I.R. (KBr): 3300, 1635, 1215 $cm^{-1}$
11) 1-[2-(4-Cyanophenoxy)phenyl]-2-methylisothiourea hydroiodide.
I.R. (KBr): 3310, 2250, 1635, 1225 $cm^{-1}$
12) 1-(2-Phenoxy-3-chlorophenyl)-2-methylisothiourea hydroiodide, mp. 164 to 166°C.
13) 1-(2-Phenoxy-4-chlorophenyl)-2-methylisothiourea hydroiodide, mp. 46 to 82°C (dec.).

14) 1-(2-Phenoxy-5-chlorophenyl)-2-methylisothiourea hydroiodide, mp. 152 to 178°C.
15) 1-(2-Phenoxy-6-chlorophenyl)-2-methylisothiourea hydroiodide, mp. 142 to 151°C.
16) 1-(2-Phenoxy-5-methylphenyl)-2-methylisothiourea hydroiodide.
I.R. (film): 3260, 1625, 1225 $cm^{-1}$
17) 1-(2-Phenoxy-6-methylphenyl)-2-methylisothiourea hydroiodide, mp. 178 to 188°C.
18) 1-(2-Phenoxy-5-hydroxyphenyl)-2-methylisothiourea hydroiodide.
I.R. (film): 3300, 3200, 1630, 1218 $cm^{-1}$
19) 1-(2-Phenoxy-5-methoxyphenyl)-2-methylisothiourea hydroiodide, mp. 124 to 129°C.
20) 1-(2-Phenoxy-5-nitrophenyl)-2-methylisothiourea hydroiodide, mp. 53 to 84°C.
21) 1-(2-Phenoxy-5-methanesulfonamidophenyl)-2-methylisothiourea hydroiodide, mp. 61 to 100°C.
22) 1-(2-Phenoxy-5-carbamoylphenyl)-2-methylisothiourea hydroiodide.
I.R. (KBr): 3350, 3200, 3150, 1685, 1660, 1650, 1270, 1225 $cm^{-1}$
23) 1-(2-Phenoxy-5-trifluoromethylphenyl)-2-methylisothiourea hydroiodide, mp. 81 to 91°C. (dec.)
24) 1-[2-(4-Chlorophenoxy)-5-chlorophenyl]-2-methylisothiourea hydroiodide.
I.R. (KBr): 3300, 3200, 3100, 1630, 1216 $cm^{-1}$
25) 1-(2-Phenylthiophenyl)-2-methylisothiourea hydroiodide, mp. 133 to 137°C.
26) 1-(2-Benzylphenyl)-2-methylisothiourea hydroiodide.
I.R. (KBr): 3320, 3180, 1635 $cm^{-1}$
27) 1-(4-Phenoxyphenyl)-2-methylisothiourea hydroiodide, mp. 181 to 185°C.
28) 1-(3-Phenoxyphenyl)-2-methylisothiourea hydroiodide.
I.R. (film): 3260, 3150, 1630, 1215
29) 1-[2-(1-Naphthyloxy)phenyl]-2-methylisothiourea hydroiodide, mp. 57 to 107°C.
30) 1-(2-Biphenylyl)-2-methylisothiourea hydroiodide.
I.R. (KBr): 3310, 1635 $cm^{-1}$
31) 1-[2-(4-Chlorophenyl)phenyl]-2-methylisothiourea hydroiodide.
I.R. (KBr): 3300, 3200, 1635 $cm^{-1}$
32) 1-[2-Phenyl-4-chlorophenyl]-2-methylisothiourea hydroiodide,
I.R. (KBr): 3300, 3180, 1635 $cm^{-1}$
33) 1-[2-Phenyl-5-chlorophenyl]-2-methylisothiourea hydroiodide.
I.R. (KBr): 3300, 3190, 1635 $cm^{-1}$
34) 1-[2-(2-Fluorophenyl)phenyl]-2-methylisothiourea hydroiodide.
I.R. (KBr): 3100, 1635 $cm^{-1}$
35) 1-[2-(2-Chlorophenyl)phenyl]-2-methylisothiourea hydroiodide.
I.R. (KBr): 3100, 1635 $cm^{-1}$
36) 1-(2-Phenyl-6-chlorophenyl)-2-methylisothiourea hydroiodide.
I.R. (KBr): 3100, 1630 $cm^{-1}$
37) 1-[4-(2,3-Dimethylphenoxy)-2,6-dichlorophenyl]-2-methylisothiourea hydroiodide, mp. 185 to 195°C.
38) 1-(2-Phenyl-3-chlorophenyl)-2-methylisothiourea hydriodide.
I.R. (KBr): 3050, 1620 $cm^{-1}$
39) 1-[2-(m-Tolyl)phenyl]-2-methylisothiourea hydriodide.
I.R. (KBr): 3050, 1620 $cm^{-1}$
40) 1-[2-(2-Nitrophenyl)phenyl]-2-methylisothiourea hydriodide.
I.R. (Film): 3080, 1625, 1520, 1350 $cm^{-1}$
41) 1-[2-(2,4-Difluorophenyl)phenyl]-2-methylisothiourea hydriodide.
I.R. (Film): 3080, 1625 $cm^{-1}$
42) 1-[2-(2,6-Difluorophenyl)phenyl]-2-methylisothiourea hydriodide.
I.R. (Film): 3260, 3080, 1620 $cm^{-1}$
43) 1-(2-Phenyl-5-methylphenyl)-2-methylisothiourea hydriodide.
I.R. (Film): 3270, 3170, 3100, 1625 $cm^{-1}$
44) 1-[2-(2-Methoxyphenyl)phenyl]-2-methylisothiourea hydriodide.
I.R. (Film): 3400, 3250, 1620 $cm^{-1}$
45) 1[2-(2-Trifluoromethylphenyl)phenyl]-2-methylisothiourea hydriodide.
I.R. (Film): 3400, 1625 $cm^{-1}$
46) 1-[2-(2-Dimethylaminophenyl)phenyl]-2-methylisothiourea hydriodide.
I.R. (Nujol): 3250, 3075, 1625 $cm^{-1}$
47) 1-[2-(3-Chloro-4-sulfamoylphenoxy)phenyl]-2-methylisothiourea hydriodide.
I.R. (Film): 3300, 1635, 1350, 1170 $cm^{-1}$

Preparation of the object compounds

Example 1

A solution of 1-(2-phenoxyphenyl)-2-methylisothiourea hydroiodide (16.3 g.) and ethylenediamine (7.6 g.) in dry ethanol (160 ml.) was refluxed for 2 hours. The reaction mixture was cooled and then evaporated under reduced pressure. After addition of ethyl acetate and water to the

residue, the resulting mixture was adjusted to pH 9 to 10 with an aqueous solution of sodium hydroxide and then shaken. The organic layer was separated, washed with water, dried over magnesium sulfate and then evaporated. The residue was recrystallized from a mixture of diisopropyl ether and ethyl acetate to give 2-(2-phenoxyphenyl)amino-2-imidazoline (6.1 g.), mp. 167 to 171°C.

Elemental analysis:

| | C | H | N |
|---|---|---|---|
| Calcd: | 71.12 | 5.97 | 16.59 |
| Found: | 71.25 | 5.96 | 16.44 |

Example 2

The following compounds were obtained according to the similar manner to that of Example 1.

1) 2-[2-(2-Chlorophenoxy)phenyl]amino-2-imidazoline, mp. 158 to 161°C.

Elemental analysis:

| | C | H | N |
|---|---|---|---|
| Calcd: | 62.61 | 4.90 | 14.60 |
| Found: | 62.74 | 4.89 | 14.45 |

2) 2-[2-(3-Chlorophenoxy)phenyl]amino-2-imidazoline, mp. 77 to 78°C.

Elemental analysis:

| | C | H | N |
|---|---|---|---|
| Calcd: | 62.61 | 4.90 | 14.60 |
| Found: | 62.45 | 4.83 | 14.58 |

3) 2-[2-(3-Chlorophenoxy)phenyl]amino-2-imidazoline fumarate, mp. 169 to 175°C.

N.M.R. (DMSO-$d_6$, $\delta$): 3.60 (4H, s), 6.47 (2H, s), 6.95—7.55 (8H, m), 9.50 (4H, broad s)

4) 2-[2-(4-Chlorophenoxy)phenyl]amino-2-imidazoline, mp. 111 to 119°C.

Elemental analysis:

| | C | H | N |
|---|---|---|---|
| Calcd: | 62.61 | 4.90 | 14.60 |
| Found: | 62.34 | 4.76 | 14.28 |

5) 2-[2-(2,6-Dichlorophenoxy)phenyl]amino-2-imidazoline, mp. 182 to 185°C.

Elemental analysis:

| | C | H | N |
|---|---|---|---|
| Calcd: | 55.92 | 4.07 | 13.04 |
| Found: | 56.32 | 3.94 | 12.83 |

6) 2-[2-(3,4-Dichlorophenoxy)phenyl]amino-2-imidazoline fumarate, mp. 166 to 169°C.
7) 2-[2-(3,5-Dichlorophenoxy)phenyl]amino-2-imidazoline, mp. 168 to 170°C.
8) 2-[2-(o-Tolyloxy)phenyl]amino-2-imidazoline, mp. 116 to 117°C.

Elemental analysis:

| | C | H | N |
|---|---|---|---|
| Calcd: | 71.89 | 6.41 | 15.72 |
| Found: | 71.63 | 6.22 | 15.71 |

9) 2-[2-(m-Tolyloxy)phenyl]amino-2-imidazoline, mp. 85 to 86°C.

Elemental analysis:

| | C | H | N |
|---|---|---|---|
| Calcd: | 71.89 | 6.41 | 15.72 |
| Found: | 71.81 | 6.32 | 15.78 |

10) 2-[2-(p-Tolyloxy)phenyl]amino-2-imidazoline, mp. 126 to 129°C.

Elemental analysis:

| | C | H | N |
|---|---|---|---|
| Calcd: | 71.89 | 6.41 | 15.72 |
| Found: | 71.81 | 6.34 | 15.59 |

11) 2-[2-(3,4,5-Trimethoxyphenoxy)phenyl]amino-2-imidazoline, mp. 156 to 159°C.

Elemental analysis:

| | C | H | N |
|---|---|---|---|
| Calcd: | 62.96 | 6.16 | 12.24 |
| Found: | 62.68 | 6.07 | 12.15 |

12) 2-[2-(4-Fluorophenoxy)phenyl]amino-2-imidazoline, mp. 118 to 121°C.

Elemental analysis:

| | C | H | N |
|---|---|---|---|
| Calcd: | 66.41 | 5.20 | 15.49 |
| Found: | 65.93 | 5.16 | 15.41 |

13) 2-[2-(4-Bromophenoxy)phenyl]amino-2-imidazoline, mp. 68 to 70°C.

Elemental analysis:

| | C | H | N |
|---|---|---|---|
| Calcd: | 54.23 | 4.25 | 12.65 |
| Found: | 54.51 | 4.32 | 12.30 |

14) 2-[2-(4-Cyanophenoxy)phenyl]amino-2-imidazoline, mp. 161 to 163°C.

Elemental analysis:

| | C | H | N |
|---|---|---|---|
| Calcd: | 69.05 | 5.07 | 20.13 |
| Found: | 69.40 | 4.93 | 20.12 |

15) 2-(2-Phenoxy-3-chlorophenyl)amino-2-imidazoline, mp. 150°C to 158°C.

Elemental analysis:

| | C | H | N |
|---|---|---|---|
| Calcd: | 62.61 | 4.90 | 14.60 |
| Found: | 62.61 | 4.90 | 14.51 |

16) 2-(2-Phenoxy-4-chlorophenyl)amino-2-imidazoline, mp. 163 to 172°C.

Elemental analysis:

| | C | H | N |
|---|---|---|---|
| Calcd: | 62.61 | 4.90 | 14.60 |
| Found; | 62.51 | 4.83 | 14.54 |

17) 2-(2-Phenoxy-5-chlorophenyl)amino-2-imidazoline, mp. 169 to 182°C.

Elemental analysis:

| | C | H | N |
|---|---|---|---|
| Calcd: | 62.61 | 4.90 | 14.60 |
| Found: | 62.44 | 4.79 | 14.73 |

18) 2-(2-Phenoxy-6-chlorophenyl)amino-2-imidazoline, mp. 167 to 170°C.

Elemental analysis:

| | C | H | N |
|---|---|---|---|
| Calcd: | 62.61 | 4.90 | 14.60 |
| Found: | 62.58 | 4.82 | 14.57 |

19) 2-(2-Phenoxy-4-methylphenyl)amino-2-imidazoline, mp. 133 to 134°C.
20) 2-(2-Phenoxy-4-methylphenyl)amino-2-imidazoline, mp. 131 to 135°C.
21) 2-(2-Phenoxy-5-methylphenyl)amino-2-imidazoline, mp. 172 to 174°C.

Elemental analysis:

| | C | H | N |
|---|---|---|---|
| Calcd: | 71.89 | 6.41 | 15.72 |
| Found: | 71.74 | 6.30 | 15.74 |

22) 2-(2-Phenoxy-6-methylphenyl)amino-2-imidazoline, mp. 129 to 133°C.

Elemental analysis:

| | C | H | N |
|---|---|---|---|
| Calcd: | 71.89 | 6.41 | 15.72 |
| Found: | 72.02 | 6.36 | 15.72 |

23) 2-(2-Phenoxy-5-hydroxyphenyl)amino-2-imidazoline, mp. 210 to 211°C.

Elemental analysis:

| | C | H | N |
|---|---|---|---|
| Calcd: | 66.90 | 5.61 | 15.60 |
| Found: | 67.00 | 5.47 | 15.71 |

24) 2-(2-Phenoxy-5-hydroxyphenyl)amino-2-imidazoline hydrochloride, mp. 223 to 229°C.

Elemental analysis:

| | C | H | N | Cl |
|---|---|---|---|---|
| Calcd: | 58.92 | 5.27 | 13.74 | 11.60 |
| Found: | 59.25 | 5.20 | 13.42 | 11.01 |

25) 2-(2-Phenoxy-5-methoxyphenyl)amino-2-imidazoline, mp. 210 to 217°C (dec.)

Elemental analysis:

| | C | H | N |
|---|---|---|---|
| Calcd: | 67.83 | 6.05 | 14.83 |
| Found: | 68.05 | 6.05 | 14.75 |

26) 2-(2-Phenoxy-5-nitrophenyl)amino-2-imidazoline, mp. 164 to 166°C.

Elemental analysis:

| | C | H | N |
|---|---|---|---|
| Calcd: | 60.40 | 4.73 | 18.78 |
| Found: | 60.31 | 4.64 | 18.60 |

27) 2-(2-Phenoxy-5-aminophenyl)amino-2-imidazoline, mp. 153 to 157°C.
28) 2-(2-Phenoxy-5-methanesulfonamidophenyl)amino-2-imidazoline, mp. 199 to 202°C.

Elemental analysis:

| | C | H | N |
|---|---|---|---|
| Calcd: | 55.48 | 5.24 | 16.17 |
| Found: | 55.57 | 4.99 | 16.12 |

29) 2-(2-Phenoxy-5-cyanophenyl)amino-2-imidazoline, mp. 168 to 170°C.
30) 2-(2-Phenoxy-5-carbamoylphenyl)amino-2-imidazoline, mp. 236 to 238°C.
N.M.R. (DMSO-$d_6$, $\delta$): 3.28 (4H, s), 6.08 (2H, broad s), 6.78—7.97 (8H, m)
31) 2-(2-Phenoxy-5-trifluoromethylphenyl)amino-2-imidazoline, mp. 165 to 168°C.

Elemental analysis:

| | C | H | N |
|---|---|---|---|
| Calcd: | 59.81 | 4.39 | 13.08 |
| Found: | 59.83 | 4.20 | 13.11 |

32) 2-[2-(4-Chlorophenoxy)-5-chlorophenyl]amino-2-imidazoline, mp. 147 to 159°C.

Elemental analysis:

| | C | H | N |
|---|---|---|---|
| Calcd: | 55.92 | 4.07 | 13.04 |
| Found: | 56.09 | 4.00 | 12.98 |

33) 2-[2-(4-Chlorophenoxy)-5-methylphenyl]amino-2-imidazoline, mp. 132 to 135°C.
34) 2-(2-Phenylthiophenyl)amino-2-imidazoline, mp. 150 to 155°C.

Elemental analysis:

| | C | H | N |
|---|---|---|---|
| Calcd: | 66.88 | 5.61 | 15.60 |
| Found; | 66.85 | 5.64 | 15.38 |

35) 2-(2-Benzylphenyl)amino-2-imidazoline, mp. 112 to 113°C.

Elemental analysis:

| | C | H | N |
|---|---|---|---|
| Calcd: | 76.46 | 6.82 | 16.72 |
| Found: | 76.46 | 6.84 | 16.58 |

36) 2-(4-Phenoxyphenyl)amino-2-imidazoline, mp. 128 to 135°C.

Elemental analysis:

| | C | H | N |
|---|---|---|---|
| Calcd: | 71.12 | 5.97 | 16.59 |
| Found: | 70.76 | 5.99 | 16.37 |

37) 2-(4-Phenoxyphenyl)amino-2-imidazoline hydrochloride, mp. 154 to 157°C.

Elemental analysis:

| | C | H | N | Cl |
|---|---|---|---|---|
| Calcd: | 62.17 | 5.57 | 14.50 | 12.24 |
| Found: | 61.98 | 5.48 | 14.44 | 12.21 |

38) 2-(3-Phenoxyphenyl)amino-2-imidazoline, mp. 57 to 75°C.
N.M.R. (DMSO-$d_6$, $\delta$): 3.35 (4H, s), 6.40—7.44 (11H, m)
39) 2-(3-Phenoxyphenyl)amino-2-imidazoline hydrochloride, mp. 155 to 157°C.

Elemental analysis:

| | C | H | N |
|---|---|---|---|
| Calcd: | 62.17 | 5.57 | 14.50 |
| Found: | 62.41 | 5.52 | 14.45 |

40) 2-[2-(1-Naphthyloxy)phenyl]amino-2-imidazoline, mp. 129 to 136°C.

Elemental analysis:

| | C | H | N |
|---|---|---|---|
| Calcd: | 75.22 | 5.65 | 13.85 |
| Found: | 75.49 | 5.60 | 13.91 |

41) 2-(2-Biphenylyl)amino-2-imidazoline, mp. 170 to 171°C.

Elemental analysis:

| | C | H | N |
|---|---|---|---|
| Calcd: | 75.92 | 6.37 | 17.71 |
| Found: | 76.56 | 6.37 | 17.80 |

42) 2-[2-(4-Chlorophenyl)phenyl]amino-2-imidazoline, mp. 148 to 150°C.

Elemental analysis:

| | C | H | N |
|---|---|---|---|
| Calcd: | 66.30 | 5.19 | 15.46 |
| Found: | 66.32 | 4.98 | 15.38 |

43) 2-[2-Phenyl-4-chlorophenyl]amino-2-imidazoline, mp. 173 to 174°C.

Elemental analysis:

| | C | H | N |
|---|---|---|---|
| Calcd: | 66.30 | 5.19 | 15.46 |
| Found: | 66.42 | 4.87 | 15.38 |

44) 2-[2-Phenyl-5-chlorophenyl]amino-2-imidazoline, mp. 160—162°C.

Elemental analysis:

| | C | H | N |
|---|---|---|---|
| Calcd: | 66.30 | 5.19 | 15.46 |
| Found: | 66.49 | 4.91 | 15.39 |

45) 2-(2-Phenoxy-5-dimethylaminophenyl)amino-2-imidazoline, mp. 164 to 168°C.

46) 2-[2-(2-Fluorophenyl)phenyl]amino-2-imidazoline, mp. 122 to 124°C.

Elemental analysis:

| | C | H | N |
|---|---|---|---|
| Calcd: | 70.57 | 5.53 | 16.46 |
| Found: | 70.79 | 5.44 | 16.54 |

47) 2-[2-(2-Chlorophenyl)phenyl]amino-2-imidazoline, mp. 150 to 152°C.

Elemental analysis:

| | C | H | N |
|---|---|---|---|
| Calcd: | 66.30 | 5.19 | 15.46 |
| Found: | 66.41 | 5.03 | 15.35 |

48) 2-(2-Phenyl-6-chlorophenyl)amino-2-imidazoline, mp. 130 to 135°C.

Elemental analysis:

| | C | H | N |
|---|---|---|---|
| Calcd: | 66.30 | 5.19 | 15.46 |
| Found: | 65.32 | 5.25 | 15.21 |

($H_2O$ : 2.35 %)

49) 2-[4-(2,3-Dimethylphenoxy)-2,6-dichlorophenyl]amino-2-imidazoline, mp. 176 to 179°C.

Elemental analysis:

| | C | H | N |
|---|---|---|---|
| Calcd: | 58.30 | 4.89 | 12.00 |
| Found: | 57.95 | 4.79 | 11.90 |

50) 2-[2-(3-Fluorophenyl)phenyl]amino-2-imidazoline, mp. 143 to 145°C.

Elemental analysis:

| | C | H | N |
|---|---|---|---|
| Calcd: | 70.57 | 5.53 | 16.46 |
| Found: | 70.50 | 5.20 | 16.39 |

51) 2-[2-(o-Tolyl)phenyl]amino-2-imidazoline, mp. 145 to 147°C.

Elemental analysis:

| | C | H | N |
|---|---|---|---|
| Calcd: | 76.46 | 6.82 | 16.72 |
| Found: | 76.23 | 6.70 | 16.73 |

52) 2-[2-(p-Tolyl)phenyl]amino-2-imidazoline, mp. 175 to 178°C.

Elemental analysis:

| | C | H | N |
|---|---|---|---|
| Calcd: | 76.46 | 6.82 | 16.72 |
| Found: | 76.17 | 6.84 | 16.48 |

53) 2-[2-(4-Fluorophenyl)phenyl]amino-2-imidazoline, mp. 174 to 176°C.

Elemental analysis:

| | C | H | N |
|---|---|---|---|
| Calcd: | 70.57 | 5.53 | 16.46 |
| Found: | 70.27 | 5.22 | 16.28 |

54) 2-(2-Phenoxy-5-sulfamoylphenyl)amino-2-imidazoline, mp. 174 to 178°C.

Elemental analysis:

| | C | H | N |
|---|---|---|---|
| Calcd: | 54.20 | 4.85 | 16.86 |
| Found: | 53.94 | 4.77 | 16.27 |

55) 2-(2-Phenoxy-5-morpholinophenyl)amino-2-imidazoline, mp. 167 to 168°C.

Elemental analysis:

| | C | H | N |
|---|---|---|---|
| Calcd: | 67.43 | 6.55 | 16.56 |
| Found: | 67.11 | 6.24 | 16.32 |

56) 2-(3-Biphenylyl)amino-2-imidazoline, mp. 165 to 167°C.

Elemental analysis:

| | C | H | N |
|---|---|---|---|
| Calcd: | 75.92 | 6.37 | 17.71 |
| Found: | 75.85 | 6.25 | 17.68 |

57) 2-[2-(4-Chlorophenoxy)phenyl]amino-2-imidazoline sulfate, mp. 167 to 169°C.

Elemental analysis:

| | C | H | N | S |
|---|---|---|---|---|
| Calcd: | 46.69 | 4.18 | 10.89 | 8.31 |
| Found: | 46.67 | 4.00 | 10.87 | 8.29 |

58) 2-(2-Phenyl-3-chlorophenyl)amino-2-imidazoline, mp. 165 to 167°C.

Elemental analysis:

| | C | H | N |
|---|---|---|---|
| Calcd: | 66.30 | 5.19 | 15.46 |
| Found: | 66.73 | 5.00 | 15.58 |

59) 2-[2-(4-Dimethylaminophenoxy)phenyl]amino-2-imidazoline, mp. 143 to 148°C.
60) 2-[2-Phenoxy-5-(1-pyrrolidinyl)phenyl]amino-2-imidazoline, mp. 203 to 208°C.
61) 2-[2-(m-Tolyl)phenyl]amino-2-imidazoline, mp. 150 to 152°C.

Elemental analysis:

| | C | H | N |
|---|---|---|---|
| Calcd: | 76.46 | 6.82 | 16.72 |
| Found: | 76.88 | 6.86 | 16.71 |

62) 2-[2-(2-Nitrophenyl)phenyl]amino-2-imidazoline, mp. 185 to 188°C.

Elemental analysis:

| | C | H | N |
|---|---|---|---|
| Calcd: | 63.82 | 5.00 | 19.85 |
| Found: | 63.73 | 4.85 | 19.88 |

63) 2-[2-(2,4-Difluorophenyl)phenyl]amino-2-imidazoline mp. 131 to 132°C.

Elemental analysis:

| | C | H | N |
|---|---|---|---|
| Calcd: | 65.92 | 4.79 | 15.38 |
| Found: | 66.01 | 4.62 | 15.40 |

64) 2-[2-Phenoxy-5-(N,N-dimethylsulfamoyl)phenyl]amino-2-imidazoline, mp. 149°C.
65) 2-[2-(2,5-Difluorophenyl)phenyl]amino-2-imidazoline, mp. 154 to 156°C.

Elemental analysis:

| | C | H | N |
|---|---|---|---|
| Calcd: | 65.92 | 4.79 | 15.38 |
| Found: | 66.20 | 4.53 | 15.43 |

66) 2-(2-Phenyl-5-methylphenyl)amino-2-imidazoline, mp. 177 to 180°C.

Elemental analysis:

| | C | H | N |
|---|---|---|---|
| Calcd: | 76.46 | 6.82 | 16.72 |
| Found: | 77.01 | 6.79 | 16.77 |

67) 2-[2-(2-Aminophenyl)phenyl]amino-2-imidazoline, mp. 177 to 179°C.
68) 2-[2-(2-Methoxyphenyl)phenyl]amino-2-imidazoline, mp. 150 to 153°C.

Elemental analysis:

| | C | H | N |
|---|---|---|---|
| Calcd: | 71.88 | 6.41 | 15.72 |
| Found: | 71.96 | 6.10 | 15.74 |

69) 2-[2-(2-Trifluoromethylphenyl)phenyl]amino-2-imidazoline, mp. 176 to 180°C.

Elemental analysis:

| | C | H | N |
|---|---|---|---|
| Calcd: | 62.94 | 4.62 | 13.76 |
| Found: | 63.10 | 4.66 | 13.85 |

70) 2-[2-(2-Dimethylaminophenyl)phenyl]amino-2-imidazoline, mp. 134 to 137°C.
I.R. (Nujol): 3375, 1665 $cm^{-1}$
N.M.R. ($CDCl_3$, $\delta$): 2.55 (6H, s), 3.33 (4H, s), 5.63 (2H, s), 6.97 to 7.47 (8H, m)
71) 2-[2-(3-Chloro-4-sulfamoylphenoxy)phenyl]amino-2-imidazoline, mp. 204 to 208°C.

Elemental analysis:

| | C | H | N |
|---|---|---|---|
| Calcd: | 49.11 | 4.12 | 15.27 |
| Found: | 49.05 | 4.07 | 15.33 |

## Example 3

A solution of 1-[2-(3,5-dichlorophenoxyl)phenyl]thiourea (8.7 g.) and methyl iodide (4.8 g.) in dry methanol (100 ml.) was refluxed with stirring for an hour. The reaction mixture was cooled and then evaporated under reduced pressure. After addition of methanol (100 ml.) and ethylenediamine (5.1 g.) to the residue, the mixture was refluxed with stirring for 3 hours. The reaction mixture was evaporated under reduced pressure and to the residue were added an aqueous solution of sodium carbonate and methylene chloride. The methylene chloride layer was separated, washed with water, dried over magnesium sulfate and then evaporated. The residue was recrystallized from ethyl acetate to give 2-[2-(3,5-dichlorophenoxy)phenyl]amino-2-imidazoline (5.1 g.), m.p. 168 to 170°C.

Elemental analysis:

| | C | H | N |
|---|---|---|---|
| Calcd: | 55.92 | 4.07 | 13.04 |
| Found: | 55.88 | 4.01 | 12.92 |

## Example 4

The followings compounds were obtained according to the similar manner to that of Example 3.

1) 2-(2-Phenoxyphenyl)amino-2-imidazoline, mp. 167 to 171°C.
2) 2-[2-(2-Chlorophenoxy)phenyl]amino-2-imidazoline, mp. 158 to 161°C.
3) 2-[2-(3-Chlorophenoxy)phenyl]amino-2-imidazoline, mp. 77 to 78°C.
4) 2-[2-(3-Chlorophenoxy)phenyl]amino-2-imidazoline fumarate, mp. 169 to 175°C.
5) 2-[2-(4-Chlorophenoxy)phenyl]amino-2-imidazoline, mp. 111 to 119°C.
6) 2-[2-(2,6-Dichlorophenoxy)phenyl]amino-2-imidazoline, mp. 182 to 185°C.
7) 2-[2-(3,4-Dichlorophenoxy)phenyl]amino-2-imidazoline fumarate, mp. 166 to 169°C.

Elemental analysis:

| | C | H | N |
|---|---|---|---|
| Calcd: | 52.07 | 3.91 | 9.59 |
| Found: | 52.22 | 3.80 | 9.61 |

8) 2-[2-(o-Tolyloxy)phenyl]amino-2-imidazoline, mp. 116 to 117°C.
9) 2-[2-(m-Tolyloxy)phenyl]amino-2-imidazoline, mp. 85 to 86°C.
10) 2-[2-(p-Tolyloxy)phenyl]amino-2-imidazoline, mp. 126 to 129°C.
11) 2-[2-(3,4,5-Trimethoxyphenoxy)phenyl]amino-2-imidazoline, mp. 156 to 159°C.
12) 2-[2-(4-Fluorophenoxy)phenyl]amino-2-imidazoline, mp. 118 to 121°C.
13) 2-[2-(4-Bromophenoxy)phenyl]amino-2-imidazoline, mp. 68 to 70°C.
14) 2-[2-(4-Cyanophenoxy)phenyl]amino-2-imidazoline, mp. 161 to 163°C.
15) 2-(2-Phenoxy-3-chlorophenyl)amino-2-imidazoline, mp. 150 to 158°C.
16) 2-(2-Phenoxy-4-chlorophenyl)amino-2-imidazoline, mp. 163 to 172°C.
17) 2-(2-Phenoxy-5-chlorophenyl)amino-2-imidazoline, mp. 169 to 182°C.
18) 2-(2-Phenoxy-6-chlorophenyl)amino-2-imidazoline, mp. 167 to 170°C.
19) 2-(2-Phenoxy-3-methylphenyl)amino-2-imidazoline, mp. 133 to 134°C.

Elemental analysis:

| | C | H | N |
|---|---|---|---|
| Calcd: | 71.89 | 6.41 | 15.72 |
| Found: | 71.50 | 6.38 | 15.54 |

20) 2-(2-Phenoxy-4-methylphenyl)amino-2-imidazoline, mp. 131 to 135°C.

Elemental analysis:

| | C | H | N |
|---|---|---|---|
| Calcd: | 71.89 | 6.41 | 15.72 |
| Found: | 72.06 | 6.41 | 15.55 |

21) 2-(2-Phenoxy-5-methylphenyl)amino-2-imidazoline, mp. 172 to 174°C.
22) 2-(2-Phenoxy-6-methylphenyl)amino-2-imidazoline, mp. 129 to 133°C.
23) 2-(2-Phenoxy-5-hydroxyphenyl)amino-2-imidazoline, mp. 210 to 211°C.
24) 2-(2-Phenoxy-5-hydroxyphenyl)amino-2-imidazoline hydrochloride, mp. 223 to 229°C.
25) 2-(2-Phenoxy-5-methoxyphenyl)amino-2-imidazoline, mp. 210 to 217°C (dec.).
26) 2-(2-Phenoxy-5-nitrophenyl)amino-2-imidazoline, mp. 164 to 166°C.
27) 2-(2-Phenoxy-5-aminophenyl)amino-2-imidazoline, mp. 153 to 157°C.
28) 2-(2-Phenoxy-5-methanesulfonamidophenyl)amino-2-imidazoline, mp. 199 to 202°C.
29) 2-(2-Phenoxy-5-cyanophenyl)amino-2-imidazoline, mp. 168 to 170°C.

Elemental analysis:

| | C | H | N |
|---|---|---|---|
| Calcd: | 69.05 | 5.07 | 20.13 |
| Found: | 68.98 | 4.70 | 19.81 |

30) 2-(2-Phenoxy-5-carbamoylphenyl)amino-2-imidazoline, mp. 236 to 238°C.
31) 2-(2-Phenoxy-5-trifluoromethylphenyl)amino-2-imidazoline, mp. 165 to 168°C.
32) 2-[2-(4-Chlorophenoxy)-5-chlorophenyl]amino-2-imidazoline, mp. 147 to 159°C.
33) 2-[2-(4-Chlorophenoxy)-5-methylphenyl]amino-2-imidazoline, mp. 132 to 135°C.

Elemental analysis:

| | C | H | N |
|---|---|---|---|
| Calcd: | 63.68 | 5.34 | 13.93 |
| Found: | 63.51 | 5.30 | 13.89 |

34) 2-(2-Phenylthiophenyl)amino-2-imidazoline, mp. 150 to 155°C.
35) 2-(2-Benzylphenyl)amino-2-imidazoline, mp. 112 to 113°C.
36) 2-(4-Phenoxyphenyl)amino-2-imidazoline, mp. 128 to 135°C.
37) 2-(4-Phenoxyphenyl)amino-2-imidazoline hydrochloride, mp. 154 to 157°C.

38) 2-(3-Phenoxyphenyl)amino-2-imidazoline, mp. 57 to 75°C.
39) 2-(3-Phenoxyphenyl)amino-2-imidazoline hydrochloride, mp. 155 to 157°C.
40) 2-[2-(1-Naphthyloxy)phenyl]amino-2-imidazoline, mp. 129 to 136°C.
41) 2-(2-Biphenylyl)amino-2-imidazoline, mp. 170 to 171°C.
42) 2-[2-(4-Chlorophenyl)phenyl]amino-2-imidazoline, mp. 148 to 150°C.
43) 2-[2-Phenyl-4-chlorophenyl]amino-2-imidazoline, mp. 173 to 174°C.
44) 2-[2-Phenyl-5-chlorophenyl]amino-2-imidazoline, mp. 160 to 162°C.
45) 2-(2-Phenoxy-5-dimethylaminophenyl)amino-2-imidazoline, mp. 164 to 168°C.

Elemental analysis:

| | C | H | N |
|---|---|---|---|
| Calcd: | 68.89 | 6.80 | 18.91 |
| Found: | 68.81 | 6.74 | 18.72 |

46) 2-[2-(2-Fluorophenyl)phenyl]amino-2-imidazoline, mp. 122 to 124°C.
47) 2-[2-(2-Chlorophenyl)phenyl]amino-2-imidazoline, mp. 150 to 152°C.
48) 2-(2-Phenyl-6-chlorophenyl)amino-2-imidazoline, mp. 130 to 135°C.
49) 2-[4-(2,3-Dimethylphenoxy)-2,6-dichlorophenyl]amino-2-imidazoline, mp. 176 to 179°C.
50) 2-[2-(3-Fluorophenyl)phenyl]amino-2-imidazoline, mp. 143 to 145°C.
51) 2-[2-(o-Tolyl)phenyl]amino-2-imidazoline, mp. 145 to 147°C.
52) 2-[2-(p-Tolyl)phenyl]amino-2-imidazoline, mp. 175 to 178°C.
53) 2-[2-(4-Fluorophenyl)phenyl]amino-2-imidazoline, mp. 174 to 176°C.
54) 2-(2-Phenoxy-5-sulfamoylphenyl)amino-2-imidazoline, mp. 174 to 178°C.
55) 2-(2-Phenoxy-5-morpholinophenyl)amino-2-imidazoline, mp. 167 to 168°C.
56) 2-(3-Biphenylyl)amino-2-imidazoline, mp. 165 to 167°C.
57) 2-[2-(4-Chlorophenoxy)phenyl]amino-2-imidazoline sulfate, mp. 167 to 169°C.
58) 2-(2-Phenyl-3-chlorophenyl)amino-2-imidazoline, mp. 165 to 167°C.
59) 2-[2-(4-Dimethylaminophenoxy)phenyl]amino-2-imidazoline, mp. 143 to 148°C.

Elemental analysis:

| | C | H | N |
|---|---|---|---|
| Calcd: | 68.89 | 6.80 | 18.91 |
| Found: | 68.72 | 6.77 | 18.92 |

60) 2-[2-Phenoxy-5-(1-pyrrolidinyl)phenyl]amino-2-imidazoline, mp. 203 to 208°C.

Elemental analysis:

| | C | H | N |
|---|---|---|---|
| Calcd: | 70.78 | 6.88 | 17.38 |
| Found: | 70.61 | 6.83 | 17.23 |

61) 2-[2-(m-Tolyl)phenyl]amino-2-imidazoline, mp. 150 to 152°C.
62) 2-[2-(2-Nitrophenyl)phenyl]amino-2-imidazoline, mp. 185 to 188°C.
63) 2-[2-(2,4-Difluorophenyl)phenyl]amino-2-imidazoline, mp. 131 to 132°C.
64) 2-[2-Phenoxy-5-(N,N-dimethylsulfamoyl)phenyl]amino-2-imidazoline, mp. 149°C.

Elemental analysis:

| | C | H | N |
|---|---|---|---|
| Calcd: | 56.65 | 5.59 | 15.55 |
| Found: | 56.91 | 5.60 | 15.52 |

65) 2-[2-(2,6-Difluorophenyl)phenyl]amino-2-imidazoline, mp. 154 to 156°C.
66) 2-(2-Phenyl-5-methylphenyl)amino-2-imidazoline, mp. 177 to 180°C.
67) 2-[2-(2-Aminophenyl)phenyl]amino-2-imidazoline, mp. 177 to 179°C.
68) 2-[2-(2-Methoxyphenyl)phenyl]amino-2-imidazoline, mp. 150 to 153°C.
69) 2-[2-(2-Trifluoromethylphenyl)phenyl]amino-2-imidazoline, mp. 176 to 180°C.
70) 2-[2-(2-Dimethylaminophenyl)phenyl]amino-2-imidazoline, mp. 134 to 137°C.
71) 2-[2-(3-Chloro-4-sulfamoylphenoxy)phenyl]amino-2-imidazoline, mp. 204 to 208°C.

### Example 5

A solution of 2-chloro-2-imidazoline sulfate (1.0 g.) in 5% aqueous solution of sodium hydroxide

(10 ml.) was extracted with methylene chloride (5 ml. x 4). The extracts were combined, dried over magnesium sulfate and filtered for removal of magnesium sulfate. To the methylene chloride solution, 2-(3,4,5-trimethoxyphenoxy)-aniline (0.91 g.) was added. The mixture was stirred for 42 hours at ambient temperature. The reaction mixture was filtered and the filtrate was concentrated under reduced pressure. The concentrate was dissolved in dil. hydrochloric acid, washed with ethyl acetate twice, made basic with an aqueous solution of sodium carbonate and then extracted with methylene chloride. The extract was washed with a saturated aqueous solution of sodium chloride, dried over magnesium sulfate and then evaporated under reduced pressure. The residue was recrystallized from a mixture of ethyl acetate and ethanol to give a white powder of 2-[2-(3,4,5-trimethoxyphenyl)-phenyl]amino-2-imidazoline (0.3 g.).

mp. 156 to 159°C.

I.R. (Nujol): 3410, 3140, 1670, 1225, 1200, 1125 $cm^{-1}$

N.M.R. (DMSO-$d_6$, $\delta$): 3.28 (4H, s), 3.62 (3H, s), 3.68 (6H, s), 5.80 (2H, broad s), 6.22 (2H, s), 6.80—7.23 (4H, m).

Example 6

The following compounds were obtained according to the similar manner to that of Example 5.

1) 2-(2-Phenoxyphenyl)amino-2-imidazoline, mp. 167 to 171°C.
2) 2-[2-(2-Chlorophenoxy)phenyl]amino-2-imidazoline, mp. 158 to 161°C.
3) 2-[2-(3-Chlorophenoxy)phenyl]amino-2-imidazoline, mp. 77 to 78°C.
4) 2-[2-(3-Chlorophenoxy)phenyl]amino-2-imidazoline fumarate, mp. 169 to 175°C.
5) 2-[2-(4-Chlorophenoxy)phenyl]amino-2-imidazoline, mp. 111 to 119°C.
6) 2-[2-(2,6-Dichlorophenoxy)phenyl]amino-2-imidazoline, mp. 182 to 185°C.
7) 2-[2-(3,4-Dichlorophenoxy)phenyl]amino-2-imidazoline fumarate, mp. 166 to 169°C.
8) 2-[2-(3,5-Dichlorophenoxy)phenyl]amino-2-imidazoline, mp. 168 to 170°C.
9) 2-[2-(o-Tolyloxy)phenyl]amino-2-imidazoline, mp. 116 to 117°C.
10) 2-[2-(m-Tolyloxy)phenyl]amino-2-imidazoline, mp. 85 to 86°C.
11) 2-[2-(p-Tolyloxy)phenyl]amino-2-imidazoline, mp. 126 to 129°C.
12) 2-[2-(4-Fluorophenoxy)phenyl]amino-2-imidazoline, mp. 118 to 121°C.
13) 2-[2-(4-Bromophenoxy)phenyl]amino-2-imidazoline, mp. 68 to 70°C.
14) 2-[2-(4-Cyanophenoxy)phenyl]amino-2-imidazoline, mp. 161 to 163°C.
15) 2-(2-Phenoxy-3-chlorophenyl)amino-2-imidazoline, mp. 150 to 158°C.
16) 2-(2-Phenoxy-4-chlorophenyl)amino-2-imidazoline, mp. 163 to 172°C.
17) 2-(2-Phenoxy-5-chlorophenyl)amino-2-imidazoline, mp. 169 to 182°C.
18) 2-(2-Phenoxy-6-chlorophenyl)amino-2-imidazoline, mp. 167 to 170°C.
19) 2-(2-Phenoxy-3-methylphenyl)amino-2-imidazoline, mp. 133 to 134°C.
20) 2-(2-Phenoxy-4-methylphenyl)amino-2-imidazoline, mp. 131 to 135°C.
21) 2-(2-Phenoxy-5-methylphenyl)amino-2-imidazoline, mp. 172 to 174°C.
22) 2-(2-Phenoxy-6-methylphenyl)amino-2-imidazoline, mp. 129 to 133°C.
23) 2-(2-Phenoxy-5-hydroxyphenyl)amino-2-imidazoline, mp. 210 to 211°C.
24) 2-(2-Phenoxy-5-hydroxyphenyl)amino-2-imidazoline hydrochloride, mp. 223 to 229°C.
25) 2-(2-Phenoxy-5-methoxyphenyl)amino-2-imidazoline, mp. 210 to 217°C (dec.).
26) 2-(2-Phenoxy-5-nitrophenyl)amino-2-imidazoline, mp. 164 to 166°C.
27) 2-(2-Phenoxy-5-aminophenyl)amino-2-imidazoline, mp. 153 to 157°C.
28) 2-(2-Phenoxy-5-methanesulfonamidophenyl)amino-2-imidazoline, mp. 199 to 202°C.
29) 2-(2-Phenoxy-5-cyanophenyl)amino-2-imidazoline, mp. 168 to 170°C.
30) 2-(2-Phenoxy-5-carbamoylphenyl)amino-2-imidazoline, mp. 236 to 238°C.
31) 2-(2-Phenoxy-5-trifluoromethylphenyl)amino-2-imidazoline, mp. 165 to 168°C.
32) 2-[2-(4-Chlorophenoxy)-5-chlorophenyl]amino-2-imidazoline, mp. 147 to 159°C.
33) 2-[2-(4-Chlorophenoxy)-5-methylphenyl]amino-2-imidazoline, mp. 132 to 135°C.
34) 2-(2-Phenylthiophenyl)amino-2-imidazoline, mp. 150 to 155°C.
35) 2-(2-Benzylphenyl)amino-2-imidazoline, mp. 112 to 113°C.
36) 2-(4-Phenoxyphenyl)amino-2-imidazoline, mp. 128 to 135°C.
37) 2-(4-Phenoxyphenyl)amino-2-imidazoline hydrochloride, mp. 154 to 157°C.
38) 2-(3-Phenoxyphenyl)amino-2-imidazoline, mp. 57 to 75°C.
39) 2-(3-Phenoxyphenyl)amino-2-imidazoline hydrochloride, mp. 155 to 157°C.
40) 2-[2-(1-Naphthyloxy)phenyl]amino-2-imidazoline, mp. 129 to 136°C.
41) 2-(2-Biphenylyl)amino-2-imidazoline, mp. 170 to 171°C.
42) 2-[2-(4-Chlorophenyl)phenyl]amino-2-imidazoline, mp. 148 to 150°C.
43) 2-[2-Phenyl-4-chlorophenyl]amino-2-imidazoline, mp. 173 to 174°C.
44) 2-[2-Phenyl-5-chlorophenyl]amino-2-imidazoline, mp. 160 to 162°C.
45) 2-(2-Phenoxy-5-dimethylaminophenyl)amino-2-imidazoline, mp. 164 to 168°C.
46) 2-[2-(2-Fluorophenyl)phenyl]amino-2-imidazoline, mp. 122 to 124°C.

47) 2-[2-(2-Chlorophenyl)phenyl]amino-2-imidazoline, mp. 150 to 152°C.
48) 2-(2-Phenyl-6-chlorophenyl)amino-2-imidazoline, mp. 130 to 135°C.
49) 2-[4-(2,3-Dimethylphenoxy)-2,6-dichlorophenyl]amino-2-imidazoline, mp. 176 to 179°C.
50) 2-[2-(3-Fluorophenyl)phenyl]amino-2-imidazoline, mp. 143 to 145°C.
51) 2-[2-(o-Tolyl)phenyl]amino-2-imidazoline, mp. 145 to 147°C.
52) 2-[2-(p-Tolyl)phenyl]amino-2-imidazoline, mp. 175 to 178°C.
53) 2-[2-(4-Fluorophenyl)phenyl]amino-2-imidazoline, mp. 174 to 176°C.
54) 2-(2-Phenoxy-5-sulfamoylphenyl)amino-2-imidazoline, mp. 174 to 178°C.
55) 2-(2-Phenoxy-5-morpholinophenyl)amino-2-imidazoline, mp. 167 to 168°C.
56) 2-(3-Biphenylyl)amino-2-imidazoline, mp. 165 to 167°C.
57) 2-[2-(4-Chlorophenoxy)phenyl]amino-2-imidazoline sulfate, mp. 167 to 169°C.
58) 2-(2-Phenyl-3-chlorophenyl)amino-2-imidazoline, mp. 165 to 167°C.
59) 2-[2-(4-Dimethylaminophenoxy)phenyl]amino-2-imidazoline, mp. 143 to 148°C.
60) 2-[2-Phenoxy-5-(1-pyrrolidinyl)phenyl]amino-2-imidazoline, mp. 203 to 208°C.
61) 2-[2-(m-Tolyl)phenyl]amino-2-imidazoline, mp. 150 to 152°C.
62) 2-[2-(2-Nitrophenyl)phenyl]amino-2-imidazoline, mp. 185 to 188°C.
63) 2-[2-(2,4-Difluorophenyl)phenyl]amino-2-imidazoline, mp. 131 to 132°C.
64) 2-[2-Phenoxy-5-(N,N-dimethylsulfamoyl)phenyl]amino-2-imidazoline, mp. 149°C.
65) 2-[2-(2,6-Difluorophenyl)phenyl]amino-2-imidazoline, mp. 154 to 156°C.
66) 2-(2-Phenyl-5-methylphenyl)amino-2-imidazoline, mp. 177 to 180°C.
67) 2-[2-(2-Aminophenyl)phenyl]amino-2-imidazoline, mp. 177 to 179°C.
68) 2-[2-(2-Methoxyphenyl)phenyl]amino-2-imidazoline, mp. 150 to 153°C.
69) 2-[2-(2-Trifluoromethylphenyl)phenyl]amino-2-imidazoline, mp. 176 to 180°C.
70) 2-[2-(2-Dimethylaminophenyl)phenyl]amino-2-imidazoline, mp. 134 to 137°C.
71) 2-[2-(3-Chloro-4-sulfamoylphenoxy)phenyl]amino-2-imidazoline, mp. 204 to 208°C.

Example 7

A solution of 2-(2-phenoxy-5-nitrophenyl)amino-2-imidazoline (3.0 g.) in dry methanol (240 ml.) was hydrogenated over 10% palladium-carbon (1.2 g.). The reaction mixture was filtered. The filter cake was washed with methanol. The filtrate and washing were combined and evaporated. The residue was chromatographed over Alumina, using a mixture of chloroform and methanol as an eluent. The fractions containing the desired product were collected and then evaporated. The residue was recyrstallized from a mixture of n-hexane, ethyl acetate and ethanol to give 2-(2-phenoxy-5-aminophenyl)amino-2-imidazoline (1.7 g.), mp. 153 to 157°C.

Elemental analysis:

| | C | H | N |
|---|---|---|---|
| Calcd: | 67.14 | 6.01 | 20.88 |
| Found: | 67.15 | 5.89 | 20.40 |

The following compounds were obtained according to the similar manner to that of Example 7.

(1) 2-[2-(2-Aminophenyl)phenyl]amino-2-imidazoline, mp. 177 to 179°C.

Elemental analysis:

| | C | H | N |
|---|---|---|---|
| Calcd: | 71.40 | 6.39 | 22.21 |
| Found: | 71.50 | 6.37 | 21.85 |

**Claims for the Contracting States: BE CH DE FR GB IT LI NL SE**

1. A compound of the formula:

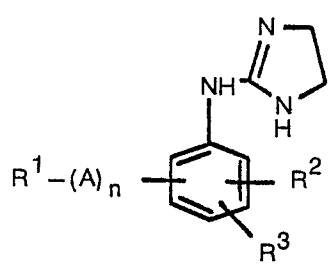

(I)

wherein $R^1$ is phenyl or naphthyl which may have 1 to 5 substituent(s) selected from halogen, 1—6C alkyl, 1—6C alkoxy, 1—6C alkanesulfonamido, mono-(or di- or tri)-halo-(1—6C)alkyl, carbamoyl, hydroxy, nitro, amino, cyano, sulfamoyl, N,N-di(1—6C)alkylsulfamoyl, and di(1—6C)alkylamino which may form a 3 to 7 membered ring with or without an oxygen or another nitrogen atom;

$R^2$ and $R^3$ are each hydrogen, halogen, 1—6C alkyl, 1—6C alkoxy, 1—6C alkanesulfonamido, mono-(or di- or tri)-halo(1—6C)alkyl, carbamoyl, hydroxy, nitro, amino, cyano, sulfamoyl, N,N-di(1—6C)alkyl-sulfamoyl, or di(1—6C)alkylamino which may form a 3 to 7-membered ring with or without an oxygen or another nitrogen atom

A is —O—, —S— or —$CH_2$—; and

n is 0 to 1, and pharmaceutically acceptable salts thereof.

2. The compound of claim 1, wherein n is 1.

3. The compound of claim 2, wherein A is —O—.

4. The compound of claim 3, which is 2-(2-phenoxyphenyl)amino-2-imidazoline.

5. The compound of claim 3, which is 2-(2-(4-chlorophenoxy)phenyl)amino-2-imidazoline or its sulfate.

6. The compound of claim 3, which is 2-(2-(4-chlorophenoxy)-5-chlorophenyl)amino-2-imidazoline.

7. The compound of claim 3, which is 2-(2-phenoxy-5-chlorophenyl)amino-2-imidazoline.

8. The compound of claim 3, which is 2-(2-phenoxy-5-methylphenyl)amino-2-imidazoline.

9. The compound of claim 3, which is 2-(2-phenoxy-3-methylphenyl)amino-2-imidazoline.

10. The compound of claim 3, which is 2-(2-phenoxy-4-methylphenyl)amino-2-imidazoline.

11. The compound of claim 3, which is 2-(2-phenoxy-5-trifluoromethylphenyl)amino-2-imidazoline.

12. The compound of claim 3, which is 2-(2-phenoxy-5-nitrophenyl)amino-2-imidazoline.

13. The compound of claim 3, which is 2-(2-(3,4,5-trimethoxyphenoxy)phenyl)amino-2-imidazoline.

14. The compound of claim 3, which is 2-(2-phenoxy-5-dimethylaminophenyl)amino-2-imidazoline.

15. The compound of claim 2, wherein A is —S—.

16. The compound of claim 15, which is 2-(2-phenylthiophenyl)amino-2-imidazoline.

17. The compound of claim 2, wherein A is —$CH_2$—.

18. The compound of claim 1, wherein n is 0.

19. The compound of claim 18, which is 2-(2-biphenyl-yl)amino-2-imidazoline.

20. The compound of claim 18, which is 2-(2-(2-chlorophenyl)phenyl)amino-2-imidazoline.

21. The compound of claim 18, which is 2-(2-(2-fluorophenyl)phenyl)amino-2-imidazoline.

22. The compound of claim 18, which is 2-(2-(4-fluorophenyl)phenyl)amino-2-imidazoline.

23. A process for preparing a compound as claimed in claim 1 which process comprises:

1) reacting a compound of the formula:

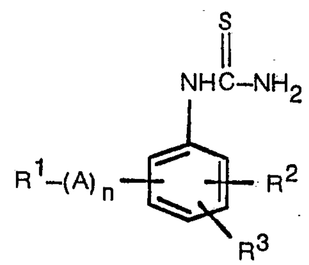

wherein $R^1$, $R^2$, $R^3$, A and n are each as defined in claim 1, or a salt thereof, with ethylenediamine or a salt thereof, to give the compound (I), or

2) reacting a compound of the formula:

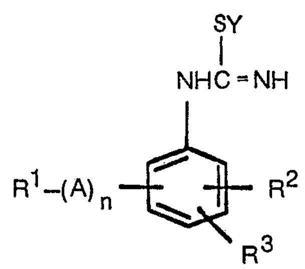

wherein $R^1$, $R^2$, $R^3$, A and n are each as defined in claim 1, and Y is 1—6C alkyl, or a salt thereof, with ethylenediamine or a salt thereof, to give the compound (I), or

3) reacting a compound of the formula:

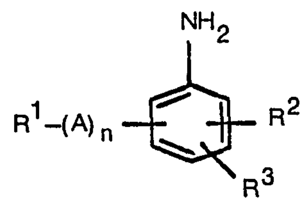

wherein $R^1$, $R^2$, $R^3$, A and n are each as defined in claim 1, or a salt thereof, with a compound of the formula:

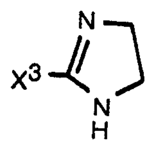

wherein $X^3$ is an acid residue, or a salt thereof, to give the compound (I), or

4) reducing a compound of the formula:

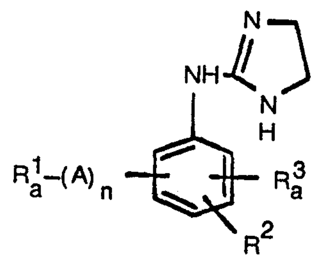

wherein $R^2$, A and n are each as defined in claim 1, and $R^1_a$ is the same as $R^1$ (in which $R^1$ is as defined in claim 1), and $R^3_a$ is hydrogen or nitro, provided that $R^1_a$ is phenyl or naphthyl substituted with nitro when $R^3_a$ is hydrogen, or a salt thereof, to give a compound of the formula:

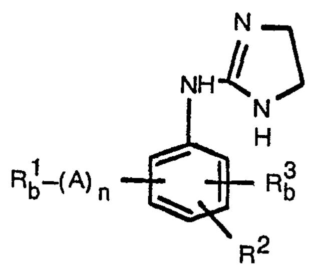

wherein $R^2$, A and n are each as defined in claim 1, $R^1_b$ is the same as $R^1$ (in which $R^1$ is as defined in claim 1), and $R^3_b$ is hydrogen or amino, provided that $R^1_b$ is phenyl or naphthyl substituted with amino when $R^3_b$ is hydrogen, or a salt thereof.

24. A pharmaceutical composition comprising a compound of claim 1 in association with a pharmaceutically acceptable, substantially non-toxic carrier or excipient.

**Claims for the Contracting State: AT**

1. A process for preparing new compounds of the formula:

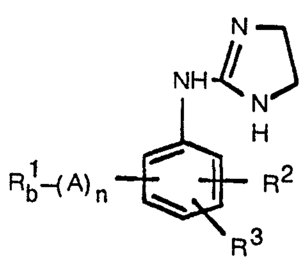

(I)

wherein $R^1$ is phenyl or naphthyl which may have 1 to 5 substituent(s) selected from halogen, 1—6C alkyl, 1—6C alkoxy, 1—6C alkanesulfonamido, mono-(or di- or tri)-halo-(1—6C)alkyl, carbamoyl, hydroxy, nitro, amino, cyano, sulfamoyl, N,N-di(1—6C)alkylsulfamoyl, and di(1—6C)alkylamino which may form a 3 to 7 membered ring with or without an oxygen or another nitrogen atom;

$R^2$ and $R^3$ are each hydrogen, halogen, 1—6C alkyl, 1—6C alkoxy, alkanesulfonamido, mono-(or di- or tri)-halo(1—6C)alkyl, carbamoyl, hydroxy, nitro, amino, cyano, sulfamoyl, N,N-di(1—6C)alkyl-sulfamoyl, or di(1—6C)alkylamino which may form a 3 to 7-membered ring with or without an oxygen or another nitrogen atom;

A is —O—, —S— or $—CH_2—$; and

n is 0 or 1, which process comprises:

1) reacting a compound of the formula:

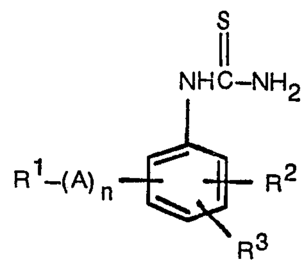

wherein $R^1$, $R^2$, $R^3$, A and n are each as defined above, or a salt thereof, with ethylenediamine or a salt thereof, to give the compound (I), or

2) reacting a compound of the formula:

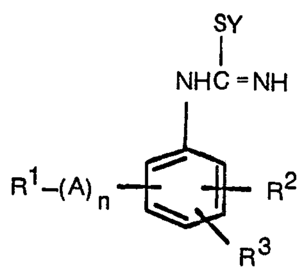

wherein $R^1$, $R^2$, $R^3$, A and n are each as defined above, and Y is 1—6C alkyl, or a salt thereof, with ethylenediamine or a salt thereof, to give the compound (I), or

3) reacting a compound of the formula:

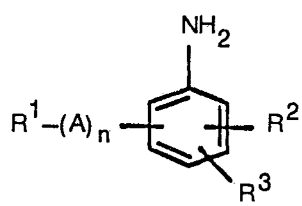

wherein $R^1$, $R^2$, $R^3$, A and n are each as defined above, or a salt thereof, with a compound of the formula:

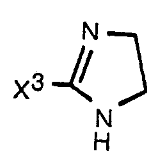

wherein $X^3$ is an acid residue, or a salt thereof, to give the compound (I), or

4) reducing a compound of the formula:

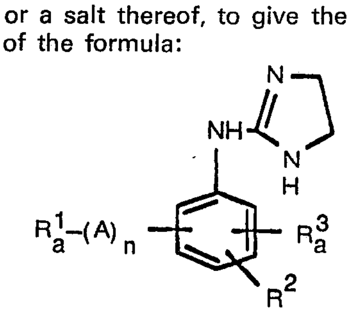

wherein $R^2$, A and n are each as defined above, and $R^1_a$ is the same as $R^1$ (in which $R^1$ is as defined above), and $R^3_a$ is hydrogen or nitro, providing that $R^1_a$ is phenyl or naphthyl substituted with nitro when $R^3_a$ is hydrogen, or a salt thereof, to give a compound of the formula:

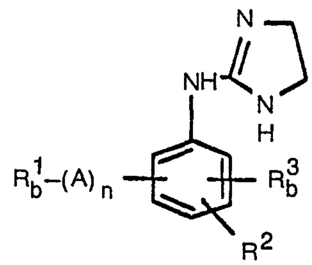

wherein $R^2$, A and n are each as defined above, $R^1_b$ is the same as $R^1$ (in which $R^1$ is as defined above), and $R^3_b$ is hydrogen or amino, provided that $R^1_b$ is phenyl or naphthyl substituted with amino when $R^3_b$ is hydrogen, or a salt thereof.

2. A process according to claim 1, which process comprises reacting a compound of the formula:

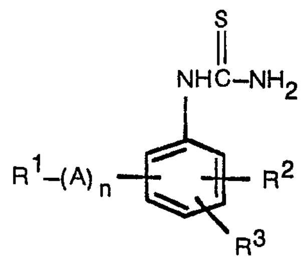

wherein $R^1$, $R^2$, $R^3$, A and n are each as defined in claim 1, or a salt thereof, with ethylenediamine or a salt thereof.

3. A process according to claim 1, which process comprises reacting a compound of the formula:

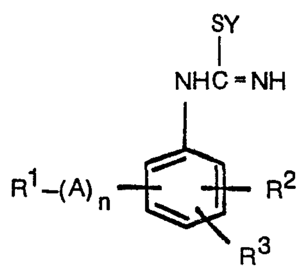

wherein $R^1$, $R^2$, $R^3$, A and n are each as defined in claim 1 and Y is 1—6C alkyl, or a salt thereof, with ethylenediamine or a salt thereof.

4. A process according to claim 1, which process comprises reacting a compound of the formula:

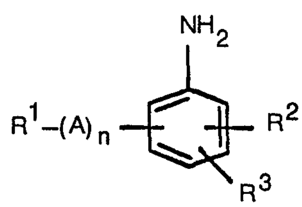

wherein $R^1$, $R^2$, $R^3$, A and n are each as defined in claim 1, or a salt thereof, with a compound of the formula:

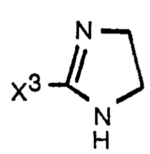

wherein $X^3$ is an acid residue, or a salt thereof.

5. A process according to claim 1, which process comprises reducing a compound of the formula:—

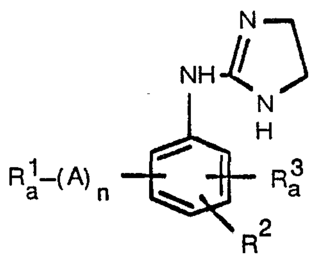

wherein $R^2$, A and n are each as defined in claim 1, and $R_a^1$ is the same as $R^1$ (in which $R^1$ is as defined in claim 1) and $R_a^3$ is hydrogen or nitro, provided that $R_a^1$ is phenyl or naphthyl substituted with nitro when $R^3$ is hydrogen, or a salt thereof, to give a compound of the formula:

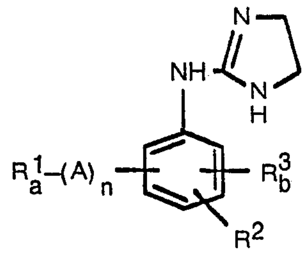

wherein $R^2$, A and n are each as defined in claim 1, $R_b^1$ is the same as $R^1$ (in which $R^1$ is as defined in claim 1) and $R_b^3$ is hydrogen or amino, provided that $R_b^1$ is phenyl or naphthyl substituted with amino when $R_b^3$ is hydrogen, or a salt thereof.

**Patentansprüche für die Vertragsstaaten: BE CH DE FR GB IT LI NL SE**

1. Eine Verbindung der Formel

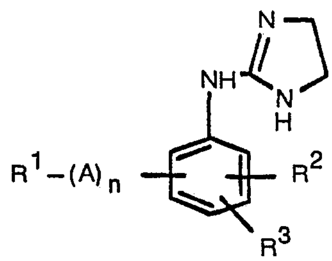
, (I)

worin $R^1$ Phenyl oder Naphthyl, die 1 bis 5 Substituenten ausgewählt aus Halogen, $C_{1-6}$-Alkyl, $C_{1-6}$-Alkoxy, $C_{1-6}$-Alkanesulfonamido, Mono- oder (Di- oder Tri)-halogen-($C_{1-6}$-)alkyl, Carbamoyl, Hydroxy, Nitro, Amino, Cyano, sulfamoyl, N,N-Di-($C_{1-6}$-)alkylsulfamoyl und Di-($C_{1-6}$-)alkylamino, welches mit oder ohne ein(em) Sauerstoff- oder ein(em) weiteren Stickstoffatom einen 3- bis 7-gliedrigen Ring bilden kann, aufweisen können, bedeutet;

$R^2$ und $R^3$ jeweils Wasserstoff, Halogen, $C_{1-6}$-Alkyl, $C_{1-6}$-Alkoxy, $C_{1-6}$-Alkansulfonamido, Mono-(oder Di- oder Tri)-halogen-($C_{1-6}$-)alkyl, Carbamoyl, Hydroxy, Nitro, Amino, Cyano, Sulfamoyl, N,N-Di-($C_{1-6}$-)alkyl-sulfamoyl oder Di-($C_{1-6}$-)alkylamino, welches, mit oder ohne ein(em) Sauerstoff- oder ein(em) weiteren Stickstoffatom einen 3- bis 7-gliedrigen Ring bilden kann, sind;

A —O—, —S— oder —$CH_2$— ist; und

n Null oder 1 ist, und deren pharmazeutisch annehmbare Salze.

2. Die Verbindung von Anspruch 1, worin n 1 ist.

3. Die Verbindung von Anspruch 2, worin A —O— bedeutet.

4. Die Verbindung von Anspruch 3, die 2-(2-Phenoxyphenyl)-amino-2-imidazolin ist.

5. Die Verbindung von Anspruch 3, die 2-[2-(4-Chlorphenoxy)phenyl]-amino-2-imidazolin oder dessen Sulfat ist.

6. Die Verbindung von Anspruch 3, die 2-[2-(4-Chlorphenoxy)-5-chlorophenyl]-amino-2-imidazolin ist.

7. Die Verbindung von Anspruch 3, die 2-(2-Phenoxy-5-chlorphenyl)-amino-2-imidazolin ist.

8. Die Verbindung von Anspruch 3, die 2-(2-Phenoxy-5-methylphenyl)-amino-2-imidazolin ist.

9. Die Verbindung von Anspruch 3, die 2-(2-Phenoxy-3-methylphenyl)-amino-2-imidazolin ist.

10. Die Verbindung von Anspruch 3, die 2-(2-Phenoxy-4-methylphenyl)-amino-2-imidazolin ist.

11. Die Verbindung von Anspruch 3, die 2-(2-Phenoxy-5-trifluormethylphenyl)-amino-2-imidazolin ist.

12. Die Verbindung von Anspruch 3, die 2-(2-Phenoxy-5-nitrophenyl)-amino-2-imidazolin ist.

13. Die Verbindung von Anspruch 3, die 2-[2-(3,4,5-Trimethoxyphenoxy))-phenyl]-amino-2-imidazolin ist.

14. Die Verbindung von Anspruch 3, die 2-(2-Phenoxy-5-dimethylaminophenyl)-amino-2-imidazolin ist.

15. Die Verbindung von Anspruch 2, worin A —S— bedeutet.

16. Die Verbindung von Anspruch 15, die 2-(2-Phenylthiophenyl)-amino-2-imidazolin ist.

17. Die Verbindung von Anspruch 2, worin A $—CH_2—$ bedeutet.

18. Die Verbindung von Anspruch 1, worin n Null ist.

19. Die Verbindung von Anspruch 18, die 2-(2-Biphenyl-yl)-amino-2-imidazolin ist.

20. Die Verbindung von Anspruch 18, die 2-[2-(2-Chlorphenyl)-phenyl]-amino-2-imidazolin ist.

21. Die Verbindung von Anspruch 18, die 2-[2-(2-Fluorphenyl)-phenyl]-amino-2-imidazolin ist.

22. Die Verbindung von Anspruch 18, die 2-[2-(4-Fluorphenyl)-phenyl]-amino-2-imidazolin ist.

23. Verfahren zum Herstellen einer Verbindung, wie in Anspruch 1 beansprucht, welches Verfahren umfaßt:

1) das Umsetzen einer Verbindung der Formel

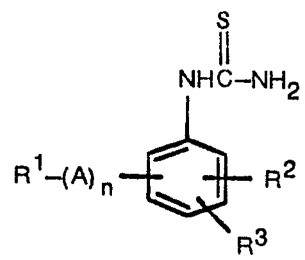
,

worin $R^1$, $R^2$, $R^3$, A und n jeweils wie in Anspruch 1 definiert sind, oder eines Salzes hievon mit Äthylendiamin oder einem Salz hievon, um die Verbindung (I) zu erhalten, oder

2) das Umsetzen einer Verbindung der Formel

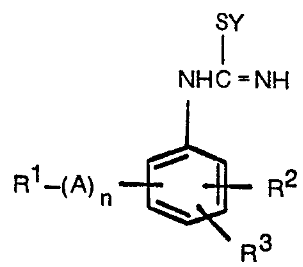
,

worin $R^1$, $R^2$, $R^3$, A und n jeweils wie in Anspruch 1 definiert sind, und Y $C_{1-6}$-Alkyl bedeutet, oder eines Salzes hievon mit Äthylendiamin oder einem Salz hievon, um die Verbindung (I) zu erhalten, oder

3) das Umsetzen einer Verbindung der Formel

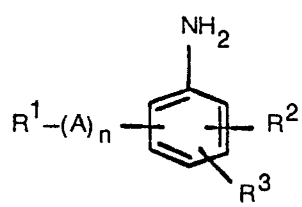
,

worin $R^1$, $R^2$, $R^3$, A und n jeweils wie in Anspruch 1 definiert sind, oder eines Salzes hievon mit einer Verbindung der Formel

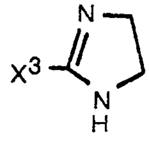
,

worin $X^3$ einen Säurerest bedeutet, oder einem Salz hievon, um die Verbindung (I) zu erhalten, oder

4) das Reduzieren einer Verbindung der Formel

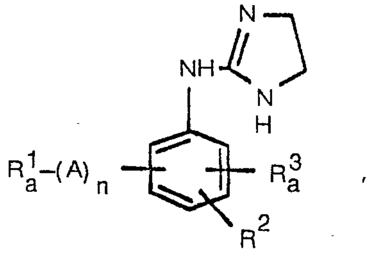

worin $R^2$, A und n jeweils wie in Anspruch 1 definiert sind, $R^1_a$ die gleiche Bedeutung wie $R^1$ hat (wobei $R^1$ wie in Anspruch 1 definiert ist) und $R^3_a$ Wasserstoff oder Nitro ist, vorausgesetzt daß $R^1_a$ Phenyl oder naphthyl substituiert mit Nitro ist, wenn $R^3_a$ Wasserstoff bedeutet, oder eines Salzes hievon, um eine Verbindung der Formel

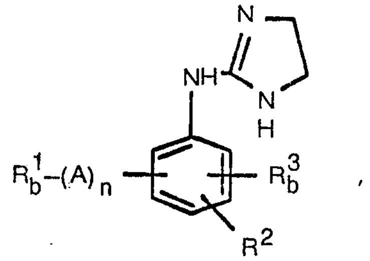

worin $R^2$, A und n jeweils wie in Anspruch 1 definiert sind, $R^1_b$ die gleiche Bedeutung wie $R^1$ hat (wobei $R^1$ wie in Anspruch 1 definiert ist) und $R^3_b$ Wasserstoff oder Amino ist, vorausgesetzt daß $R^1_b$ Phenyl oder Naphthyl suibstituiert mit Amino ist, wenn $R^3_b$ Wasserstoff bedeutet, oder ein Salz hievon zu erhalten.

24. Pharmazeutische Zusammensetzung, die eine Verbindung von Anspruch 1 in Vereinigung mit einem pharmazeutisch annehmbaren, im wesentlichen nicht-toxischen Träger oder Exzipienten umfaßt.

**Patentansprüche für den Vertragsstaat: AT**

1. Verfahren zum Herstellen von neuen Verbindungen der Formel

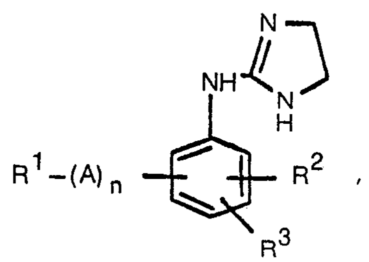

(I)

worin $R^1$ Phenyl oder Naphthyl, die 1 bis 5 Substituenten ausgewählt aus Halogen, $C_{1-6}$-Alkyl, $C_{1-6}$-Alkoxy, $C_{1-6}$-Alkansulfonamido, Mono- oder (Di- oder Tri)-halogen-($C_{1-6}$-)alkyl, Carbamoyl, Hydroxy, Nitro, Amino, Cyano, Sulfamoyl, N,N-Di-($C_{1-6}$-)alkylsulfamoyl und Di-($C_{1-6}$-)alkylamino, welches mit oder ohne ein(em) Sauerstoff- oder ein(em) weiteren Stickstoffatom einen 3- bis 7-gliedrigen Ring bilden kann, aufweisen können, bedeutet;

$R^2$ und $R^3$ jeweils Wasserstoff, Halogen, $C_{1-6}$-Alkyl, $C_{1-6}$-Alkoxy, Alkansulfonamido, Mono- (oder Di- oder Tri)-halogen-($C_{1-6}$-)alkyl, Carbamoyl, Hydroxy, Nitro, Amino, Cyano, Sulfamoyl, N,N-Di-($C_{1-6}$-)alkyl-sulfamoyl oder Di-($C_{1-6}$-)alkylamino, welches mit oder ohne ein(em) Sauerstoff- oder ein(em) weiteren Stickstoffatom einen 3- bis 7-gliedrigen Ring bilden kann, sind;

A —O—, —S— oder —$CH_2$— ist; und

n Null oder 1 ist, welches Verfahren umfaßt:

1) das Umsetzen einer Verbindung der Formel

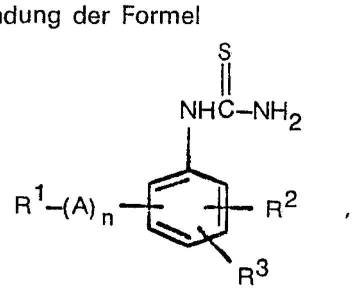

worin $R^1$, $R^2$, $R^3$, A und n jeweils wie oben definiert sind, oder eines Salzes hievon mit Äthylendiamin oder einem Salz hievon, um die Verbindung (I) zu erhalten, oder

2) das Umsetzen einer Verbindung der Formel

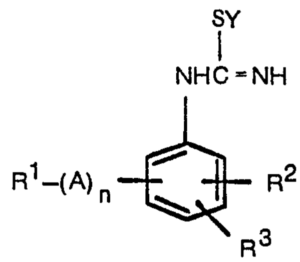

,

worin $R^1$, $R^2$, $R^3$, A und n jeweils wie oben definiert sind, und Y $C_{1-6}$-Alkyl bedeutet, oder eines Salzes hievon mit Äthylendiamin oder einem Salz hievon, um die Verbindung (I) zu erhalten, oder

3) das Umsetzen einer Verbindung der Formel

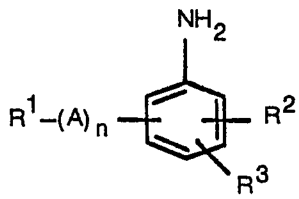

,

worin $R^1$, $R^2$, $R^3$, A und n jeweils wie oben definiert sind, oder eines Salzes hievon mit einer Verbindung der Formel

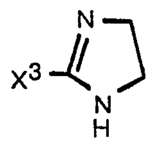

,

worin $X^3$ einen Säurerest bedeutet, oder einem Salz hievon, um die Verbindung (I) zu erhalten, oder

4) das Reduzieren einer Verbindung der Formel

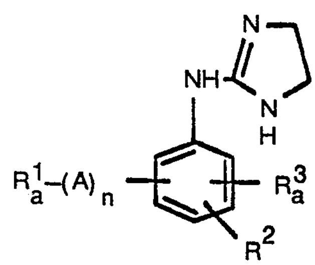

,

worin $R^2$, A und n jeweils wie oben definiert sind, $R_a^1$ die gleiche Bedeutung wie $R^1$ hat (wobei $R^1$ wie oben definiert ist) und $R_a^3$ Wasserstoff oder Nitro ist, vorausgesetzt daß $R_a^1$ Phenyl oder naphthyl substituiert mit Nitro ist, wenn $R_a^3$ Wasserstoff bedeutet, oder eines Salzes hievon, um eine Verbindung der Formel

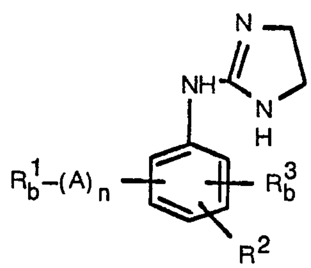

,

worin $R^2$, A und n jeweils wie oben definiert sind, $R_b^1$ die gleiche Bedeutung wie $R^1$ hat (wobei $R^1$ wie oben definiert ist) und $R_b^3$ Wasserstoff oder Amino ist, vorausgesetzt daß $R_b^1$ Phenyl oder Naphthyl substituiert mit Amino ist, wenn $R_b^3$ Wasserstoff bedeutet, oder ein Salz hievon zu erhalten.

2. Verfahren gemäß Anspruch 1, welches Verfahren das Umsetzen einer Verbindung der Formel

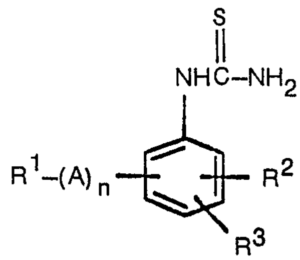
,

worin $R^1$, $R^2$, $R^3$, A und n jeweils wie in Anspruch 1 definiert sind, oder eines Salzes hievon mit Äthylendiamin oder einem Salz hievon umfaßt.

3. Verfahren gemäß Anspruch 1, welches Verfahren das Umsetzen einer Verbindung der Formel

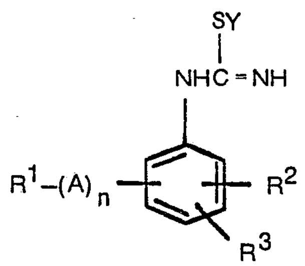
,

worin $R^1$, $R^2$, $R^3$, A und n jeweils wie in Anspruch 1 definiert sind und Y $C_{1-6}$-Alkyl ist, oder eines Salzes hievon mit Äthylendiamin oder einem Salz hievon umfaßt.

4. Verfahren gemäß Anspruch 1, welches Verfahren das Umsetzen einer Verbindung der Formel

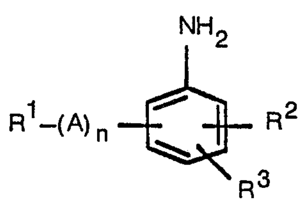
,

worin $R^1$, $R^2$, $R^3$, A und n jeweils wie in Anspruch 1 definiert sind, oder eines Salzes hievon mit einer Verbindung der Formel

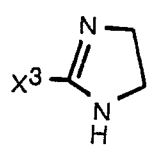
,

worin $X^3$ ein Säurerest ist, oder einem Salz hievon umfaßt.

5. Verfahren gemäß Anspruch 1, welches Verfahren das Reduzieren einer Verbindung der Formel

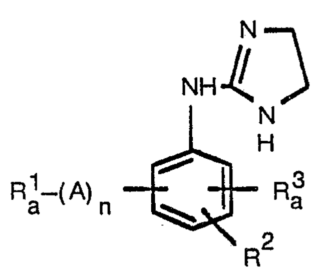
,

worin $R^2$, A und n jeweils wie in Anspruch 1 definiert sind und $R^1_a$ die gleiche bedeutung wie $R^1$ hat (wobei $R^1$ wie in Anspruch 1 definiert ist) und $R^3_a$ Wasserstoff oder Nitro ist, vorausgesetzt daß $R^1_a$ Phenyl oder Naphthyl substituiert mit Nitro ist, wenn $R^3_a$ Wasserstoff ist, oder eines Salzes hievon, um eine Verbindung der Formel

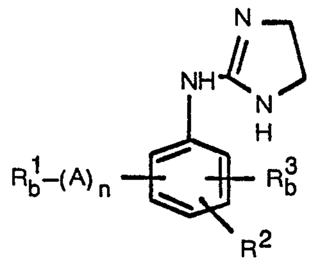

worin $R^2$, A und n jeweils wie in Anspruch 1 definiert, sind $R_b^1$ die gleiche Bedeutung wie $R^1$ hat (wobei $R^1$ wie in Anspruch 1 definiert ist) und $R_b^3$ Wasserstoff oder Amino ist, vorausgesetzt daß $R_b^1$ Phenyl oder Naphthyl substituiert mit Amino ist, wenn $R_b^3$ Wasserstoff bedeutet, oder ein Salz hievon zu erhalten.

**Revendications pour les Etats contractants: BE CH DE FR GB IT LI NL SE**

1. Composé de formule:

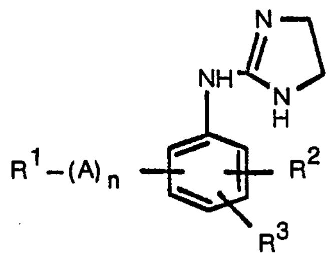

(I)

dans laquelle $R^1$ est un groupe phényle ou naphtyle qui peut comporter un à cinq substituant(s) choisi(s) parmi un atome d'hahogène, un groupe alkyle ayant 1 à 6 atomes de carbone, un groupe alcoxy ayant 1 à 6 atomes de carbone, un groupe alcanesulfonamido ayant 1 à 6 atomes de carbone, un groupe mono-(ou di- ou tri-) halogéno-alkyle (ayant 1 à 6 atomes de carbone), un groupe carbamoyle, hydroxyle, nitro, amino, cyano, sulfamoyle, N,N-dialkylisulfamoyle (1 à 6 atomes de carbone) et dialkylamino (1 à 6 atomes de carbone), qui peut former un cycle ayant trois à sept chaînons avec ou sans un atome d'oxygène ou un autre atome d'azote;

$R^2$ et $R^3$ sont chacun un atome d'hydrogène ou d'halogène, un groupe alkyle ayant 1 à 6 atomes de carbone, alcoxy ayant 1 à 6 atomes de carbone, alcanesulfonamido ayant 1 à 6 atomes de carbone, mono-(ou di- ou tri-) halogéno-alkyle (ayant 1 à 6 atomes de carbone), carbamoyle, hydroxyle, nitro, amino, cyano, sulfamoyle, N,N-dialkylsulfamoyle (1 à 6 atomes de carbone) ou dialkylamino (1 à 6 atomes de carbone), qui peut former un noyau ayant trois à sept chaînons avec ou sans un atome d'oxygène ou un autre atome d'azote;

A représente —O—, —S— ou $—CH_2—$; et

n vaut 0 ou 1, et leurs sels pharmaceutiquement acceptables.

2. Composé selon la revendication 1, dans lequel n vaut 1.

3. Composé selon la revendication 2, dans lequel A représente —O—.

4. Composé selon la revendication 3, qui est la 2-(2-phénoxyphényl)amino-2-imidazoline.

5. Composé selon la revendication 3, qui est la 2-(2-(4-chlorophénoxy)phényl)amino-2-imidazoline ou son sulfate.

6. Composé selon la revendication 3, qui est la 2-(2-(4-chlorophénoxy)-5-chlorophényl)amino-2-imidazoline.

7. Composé selon la revendication 3, qui est la 2-(2-phénoxy-5-chlorophényl)amino-2-imidazoline.

8. Composé selon la revendication 3, qui est la 2-(2-phénoxy-5-méthylphényl)amino-2-imidazoline.

9. Composé selon la revendication 3, qui est la 2-(2-phénoxy-3-méthylphényl)amino-2-imidazoline.

10. Composé selon la revendication 3, qui est la 2-(2-phénoxy-4-méthylphényl)amino-2-imidazoline.

11. Composé selon la revendication 3, qui est la 2-(2-phénoxy-5-trifluorométhylphényl)amino-2-imidazoline.

12. Composé selon la revendication 3, qui est la 2-(2-phénoxy-5-nitrophényl)amino-2-imidazoline.

13. Composé selon la revendication 3, qui est la 2-(2-(3,4,5-triméthoxyphénoxy)phényl)amino-2-imidazoline.

14. Composé selon la revendication 3, qui est la 2-(2-phénoxy-5-diméthylaminophényl)amino-2-imidazoline.

15. Composé selon la revendication 2, dans lequel A represénte —S—.

16. Composé selon la revendication 15, qui est la 2-(2-phénylthiophényl)amino-2-imidazoline.

17. Composé selon la revendication 2, dans lequel A représente —$CH_2$—.

18. Composé selon la revendication 1, dans lequel n est nul.

19. Composé selon la revendication 18, qui est la 2-(2-biphénylyl)amino-2-imidazoline.

20. Composé selon la revendication 18, qui est la 2-(2-(2-chlorophényl)phényl)amino-2-imidazoline.

21. Composé selon la revendication 18, qui est la 2-(2-(2-fluorophényl)phényl)amino-2-imidazoline.

22. Composé selon la revendication 18, qui est la 2-(2-(4-fluorophényl)phényl)amino-2-imidazoline.

23. Procédé pour préparer un composé tel que revendiqué à la revendication 1, procédé qui comprend:

1) la réaction d'un composé de formule:

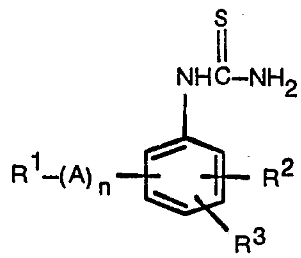

dans laquelle $R^1$, $R^2$, $R^3$, A et n sont chacun comme défini à la revendication 1, ou un sel de ce composé, avec l'éthylènediamine ou un de ses sels, pour obtenir le composé (I), ou

2) la réaction d'un composé de formule:

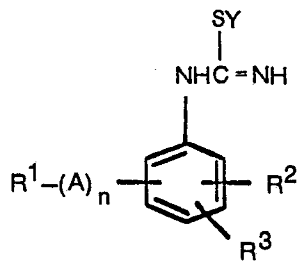

dans laquelle $R^1$, $R^2$, $R^3$, A et n sont chacun comme défini à la revendication 1, et Y représente un groupe alkyle ayant 1 à 6 atomes de carbone ou un de ses sels, avec l'éthylènediamine ou avec un de ses sels, pour obtenir le composé (I), ou

3) la réaction d'un composé de formule:

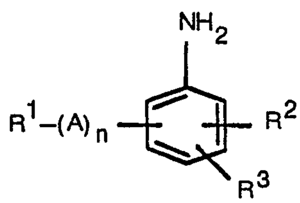

dans laquelle $R^1$, $R^2$, $R^3$, A et n sont chacun comme défini à la revendication 1) ou un de ses sels, avec un composé de formule:

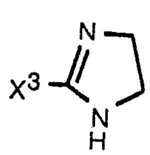

dans laquelle $X^3$ est un radical acide), ou un de ses sels, pour obtenir le composé (I), ou

4) la réduction d'un composé de formule:

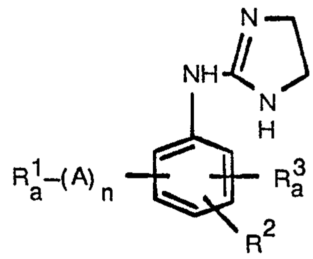

dans laquelle $R^2$, A et n sont chacun comme défini à la revendication 1, et $R_a^1$ est le même que $R^1$ ($R^1$ étant comme défini à la revendication 1), et $R_a^3$ est un atome d'hydrogène ou un groupe nitro, étant entendu que $R_a^1$ est un groupe phényle ou naphtyle substitué par un groupe nitro lorsque $R_a^3$ est un atome d'hydrogène, ou un de ses sels, pour obtenir un composé de formule:

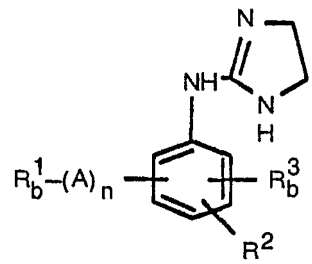

dans laquelle $R^2$, A et n sont chacun comme défini à la revendication 1, $R_b^1$ est le même que $R^1$ ($R^1$ étant comme défini à la revendication 1), et $R_b^3$ est un atome d'hydrogène ou un groupe amino, étant entendu que $R_b^1$ est un groupe phényle ou naphtyle substitué par un groupe amino lorsque $R_b^3$ est un atome d'hydrogène, ou un de ses sels.

24. Composition pharmaceutique comprenant un composé selon la revendication 1, en association avec un véhicule ou excipient pharmaceutiquement acceptable et essentiellement non toxique.

**Revendications pour l'Etat contractant: AT**

1. Procédé pour préparer de nouveaux composés de formule:

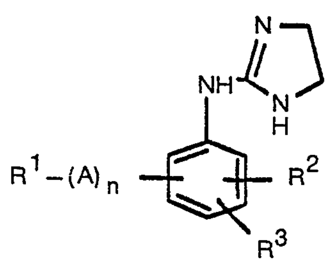
(I)

dans laquelle $R^1$ est un groupe phényle ou naphtyle qui peut comporter un à cinq substituant(s) choisi(s) parmi un atome d'halogène, un groupe alkyle ayant 1 à 6 atomes de carbone, alcoxy ayant 1 à 6 atomes de carbone, alcanesulfonamido ayant 1 à 6 atomes de carbone, mono- (ou di- ou tri-)halogénoalkyle ayant 1 à 6 atomes de carbone, carbamoyle, hydroxyle, nitro, amino, cyano, sulfamoyle, N,N-dialkylisulfamoyle (1 à 6 atomes de carbone) et dialkylamino (1 à 6 atomes de carbone), qui peut former un noyau ayant trois à sept chaînons avec ou sans un atome d'oxygène ou un autre atome d'azote;

$R^2$ et $R^3$ sont chacun un atome d'hydrogène ou d'halogène, un groupe alkyle ayant 1 à 6 atomes de carbone, alcoxy ayant 1 à 6 atomes de carbone, alcanesulfonamido, mono-(ou di- ou tri-) halogénoalkyle (1 à 6 atomes de carbone), carbamoyle, hydroxyle, nitro, amino, cyano, sulfamoyle, N,N-dialkylsulfamoyle (1 à 6 atomes de carbone dans chaque groupe alkyle), ou dialkylamino (1 à 6 atomes de carbone), qui peut former un noyau ayant trois à sept chaînons avec ou sans un atome de'oxygène ou un autre atome d'azote;

A représente —O—, —S— ou —$CH_2$—, et

n vaut 0 ou 1, procédé qui comprend:

1) la réaction d'un composé de formule:

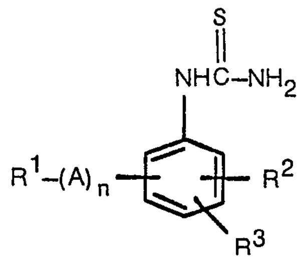

dans laquelle $R^1$, $R^2$, $R^3$, A et n sont chacun comme défini ci-dessus ou un de ses sels, avec l'éthylènediamine ou avec un de ses sels, pour obtenir le composé (I), ou

2) la réaction d'un composé de formule:

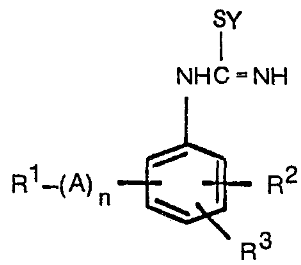

dans laquelle $R^1$, $R^2$, $R^3$, A et n sont chacun comme défini ci-dessus, et Y représente un groupe alkyle ayant 1 à 6 atomes de carbone, ou un de ses sels, avec l'éthylènediamine ou un de ses sels, pour obtenir le composé (I), ou

3) la réaction d'un composé de formule:

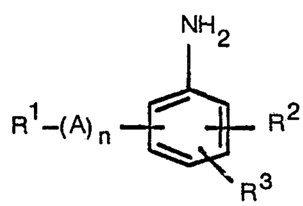

dans laquelle $R^1$, $R^2$, $R^3$, A et n sont chacun comme défini ci-dessus ou un de ses sels, avec un composé de formule:

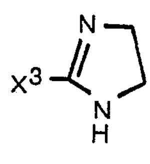

dans laquelle $X^3$ est un radical acide, ou un de ses sels, pour obtenir le composé (I), ou

4) la réduction d'un composé de formule:

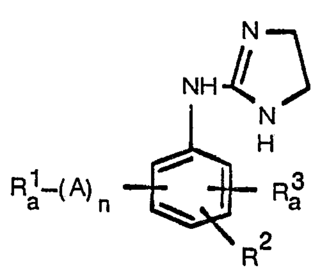

dans laquelle $R^2$, A et n sont chacun comme défini ci-dessus, et $R^1_a$ est le même que $R^1$ ($R^1$ étant comme défini ci-dessus), et $R^3_a$ est un atome d'hydrogène ou un groupe nitro, étant entendu que $R^1_a$ est un groupe phényle ou naphtyle substitué par un groupe nitro lorsque $R^3_a$ est un atome d'hydrogène ou un de ses sels, pour obtenir un composé de formule:

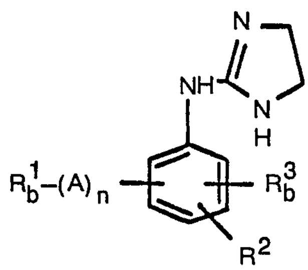

dans laquelle $R^2$, A et n sont chacun comme défini ci-dessus, $R^1_b$ est le même que $R^1$ ($R^1$ étant comme défini ci-dessus) et $R^3_b$ est un atome d'hydrogène ou un groupe amino, étant entendu que $R^1_b$ est un groupe phényle ou naphtyle substitué par un groupe amino lorsque $R^3_b$ est un atome d'hydrogène ou un de ses sels.

2. Procédé selon la revendication 1, qui comprend la réaction d'un composé de formule:

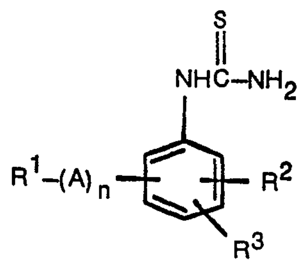

dans laquelle $R^1$, $R^2$, $R^3$, A et n sont chacun comme défini à la revendication 1, ou un de ses sels, avec l'éthylènediamine ou un de ses sels.

3. Procédé selon la revendication 1, qui comprend la réaction d'un composé de formule:

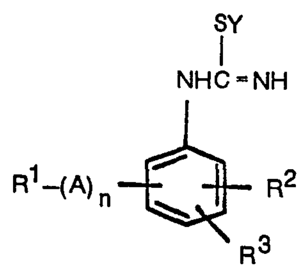

dans laquelle $R^1$, $R^2$, $R^3$, A et n sont chacun comme défini à la revendication 1, et Y est un groupe alkyle ayant 1 à 6 atomes de carbone ou un de ses sels avec l'éthylènediamine ou un de ses sels.

4. Procédé selon la revendication 1, qui comprend la réaction d'un composé de formule:

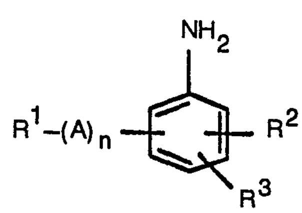

dans laquelle $R^1$, $R^2$, $R^3$, A et n sont chacun comme défini à la revendication 1 ou un de ses sels, avec un composé de formule:

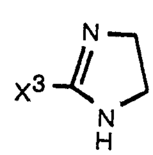

dans laquelle $X^3$ est un radical acide) ou un de ses sels.

5. Procédé selon la revendication 1, qui comprend la réduction d'un composé de formule:

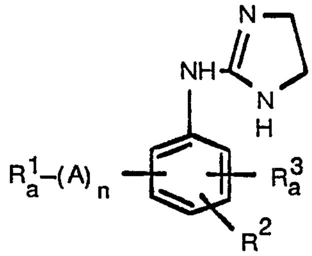

dans laquelle $R^2$, A et n sont chacun comme défini à la revendication 1, et $R_a^1$ est le même que $R^1$ ($R^1$ étant comme défini à la revendication 1) et $R_a^3$ est un atome d'hydrogène ou un groupe nitro, étant entendu que $R_a^1$ est un groupe phényle or naphtyle substitué par un groupe nitro lorsque $R^3$ est un atome d'hydrogène, ou un de ses sels, pour obtenir un composé de formule:

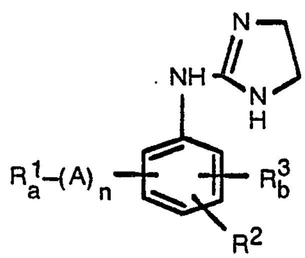

dans laquelle $R^2$, A et n sont chacun comme défini à la revendication 1, $R_b^1$ est le même que $R^1$ ($R^1$ étant comme défini à la revendication 1), et $R_b^3$ est un atome d'hydrogène ou un groupe amino, étant bien entendu que $R_b^1$ est un groupe phényle ou naphtyle substitué par un groupe amino lorsque $R_b^3$ est un atome d'hydrogène ou un de ses sels.